(19) Europäisches Patentamt
European Patent Office
Office européen des brevets

(11) EP 4 786 500 A1

(12) **EUROPEAN PATENT APPLICATION**
published in accordance with Art. 153(4) EPC

(43) Date of publication:
**05.08.2026 Bulletin 2026/32**

(21) Application number: **24872572.3**

(22) Date of filing: **27.09.2024**

(51) International Patent Classification (IPC):
***C08B 37/08*** (2006.01) ***A61K 31/728*** (2006.01)
***A61K 38/28*** (2006.01) ***A61K 45/00*** (2006.01)
***A61K 47/36*** (2006.01) ***A61L 27/20*** (2006.01)
***A61P 19/02*** (2006.01)

(52) Cooperative Patent Classification (CPC):
**A61K 31/728; A61K 38/28; A61K 45/00; A61K 47/36; A61L 27/20; A61P 19/02; C08B 37/003**

(86) International application number:
**PCT/JP2024/034816**

(87) International publication number:
**WO 2025/070791 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.09.2023 JP 2023166346**
**04.12.2023 JP 2023204796**

(71) Applicants:
- **Asahi Kasei Kabushiki Kaisha**
  **Tokyo 100-0006 (JP)**
- **Kyoto University**
  **Kyoto-shi, Kyoto 606-8501 (JP)**

(72) Inventors:
- **YABUUCHI, Kohei**
  **Tokyo 100-0006 (JP)**
- **KATSUMATA, Toru**
  **Tokyo 100-0006 (JP)**
- **SHIMOBOJI, Tsuyoshi**
  **Tokyo 100-0006 (JP)**
- **AKIYOSHI, Kazunari**
  **Kyoto-shi, Kyoto 606-8501 (JP)**

(74) Representative: **Hoffmann Eitle**
**Patent- und Rechtsanwälte PartmbB**
**Arabellastraße 30**
**81925 München (DE)**

(54) **HYALURONIC ACID DERIVATIVE, GEL OF CROSSLINKED HYALURONIC ACID DERIVATIVE, AND PHARMACEUTICAL COMPOSITION**

(57) The present invention provides: a hyaluronic acid derivative which, after having been gelled, is highly inhibited from swelling in the living body; a gel of a crosslinked hyaluronic acid derivative; and a pharmaceutical composition including the hyaluronic acid derivative or the gel of a crosslinked hyaluronic acid derivative. This hyaluronic acid derivative comprises hyaluronic acid having steryl groups introduced into at least some of the carboxyl groups contained in the glucuronic acid moieties of the hyaluronic acid and having maleimide groups introduced into at least some of either the carboxyl groups contained in the glucuronic acid moieties of the hyaluronic acid or the hydroxyl groups contained in the N-acetylglucosamine moieties thereof. This gel of a crosslinked hyaluronic acid derivative is a gel-like material obtained by chemically cross-linking the hyaluronic acid derivative with a cross-linking agent having two or more cross-linking groups.

EP 4 786 500 A1

## Description

### TECHNICAL FIELD

The present invention relates to a hyaluronic acid derivative, a cross-linked gel of a hyaluronic acid derivative obtained by cross-linking the hyaluronic acid derivative, and a pharmaceutical composition containing the same.
Priority is claimed on Japanese Patent Application No. 2023-166346, filed September 27, 2023, and Japanese Patent Application No. 2023-204796, filed December 4, 2023, the content of which is incorporated herein by reference.

### BACKGROUND ART

**In** recent years, a bio-pharmaceutical product which is a pharmaceutical product containing protein, peptide or nucleic acid as an active ingredient thereof has been developed for practical use, and the number thereof is increasing every year. The bio-pharmaceutical product can satisfy unmet medical need which cannot be satisfied by conventional low-molecular medicines. However, there are problems in terms of difficulty in absorption from the digestive tract or the mucous membrane as well as low stability in vivo and a short half-life in the blood. Thus, a bio-pharmaceutical product requires frequent administration by injection, thereby imposing excessive burdens on both patients and concerned practitioners. Therefore, there is a demand for a drug base material (base material of the sustained-release drug delivery system) which can encapsulate a bio-pharmaceutical product without impairing pharmacological activity and can release an active ingredient gradually in vivo.
From such a background, a base material of the sustained-release drug delivery system composed of a hyaluronic acid derivative having excellent safety has been proposed in Patent Document 1 and Patent Document 2. The hyaluronic acid derivative spontaneously associates in an aqueous solution, thereby effectively encapsulating a drug, particularly a bio-pharmaceutical product, while maintaining the biological activity thereof, and aggregates under a physiological saline concentration (or disperses under a physiological saline concentration), while maintaining the retention thereof in the blood favorable. It is stated that, particularly when a bio-pharmaceutical product is used as an active ingredient, the hyaluronic acid derivative may be used as a carrier which can effectively encapsulate a large amount of drug while maintaining a pharmacological activity thereof, or a sustained-release carrier or a targeting carrier, which exhibits an excellent retention property in the blood, and may also serve as a locally (such as subcutaneously) sustained-release carrier which can continuously release a drug.
Hyaluronic acid is also an important component of joints, and there is a treatment method in which hyaluronic acid is injected into the joint cavity to treat joint diseases. Although hyaluronic acid is easily injected in the treatment, a longer-term retention thereof in the joint cavity is desired. Therefore, hyaluronic acid derivatives modified with various block polymers have been proposed to achieve both ease of injection and long-term retention in Patent Document 3.

### Citation List

#### Patent Document

Patent Document 1: PCT International Publication No. WO 2010/053140
Patent Document 2: PCT International Publication No. WO 2004/046200
Patent Document 3: PCT International Publication No. WO 2003/087019

### SUMMARY OF INVENTION

#### Technical Problem

Although an injected hyaluronic acid or derivatives thereof may be gelated within the joint cavity when used to treat joint diseases, it is preferable that the volume thereof do not change significantly before and after gelation. **In** addition, it is preferable from the perspective of product design that the swelling of a cross-linked gel of a hyaluronic acid be as small as possible when used as a base material of the sustained-release drug delivery system. Furthermore, cross-linked hydrogels that allow uptake of large amounts of growth factors and proteins are preferable. However, in addition to the fact that hyaluronic acid cannot uptake proteins, it is a polysaccharide and has a high hydrophilicity, and therefore the swelling ratio thereof is high. Furthermore, hyaluronic acid derivatives described in Patent Documents 1 to 3 also did not sufficiently suppress swelling due to gelation.
The present invention has been made in view of the above-mentioned circumstances, and provides a hyaluronic

acid derivative and a cross-linked gel of the hyaluronic acid derivative that exhibit excellent swelling-suppression properties after gelation, as well as a pharmaceutical composition using these.

Solution to Problem

That is, the present invention includes the following aspects.

[1] A hyaluronic acid derivative having steryl groups introduced into at least a part of carboxyl groups in glucuronic acid moieties of the hyaluronic acid, and maleimide groups introduced into at least a part of carboxyl groups in glucuronic acid moieties or hydroxyl groups in N-acetylglucosamine moieties of the hyaluronic acid.
[2] The hyaluronic acid derivative according to [1] mentioned above, wherein the maleimide groups are introduced into the at least a part of carboxyl groups in glucuronic acid moieties of the hyaluronic acid.
[3] The hyaluronic acid derivative according to [1] or [2] mentioned above, wherein a total of an introduction rate of the steryl groups and an introduction rate of the maleimide groups relative to the hyaluronic acid derivative is less than 45%.
[4] The hyaluronic acid derivative according to any one of [1] to [3] mentioned above, wherein the introduction rate of the steryl groups relative to the hyaluronic acid derivative is 0.5% or more and 30% or less.
[5] The hyaluronic acid derivative according to any one of [1] to [4] mentioned above, wherein the introduction rate of the maleimide groups relative to the hyaluronic acid derivative is 1.0% or more and 25% or less.
[6] The hyaluronic acid derivative according to any one of [1] to [5] mentioned above, wherein the hyaluronic acid derivative has at least one repeating unit of general formula (I) shown below:

[Chemical Formula 1]

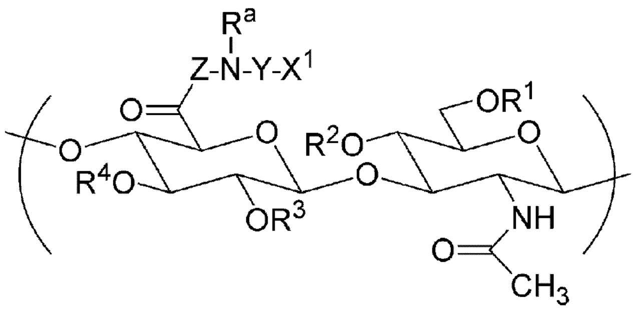
(I)

[in general formula (I), $R^1$, $R^2$, $R^3$, and $R^4$ are each independently a group selected from the group consisting of a hydrogen atom, C1-6 alkyls, a formyl and C1-6 alkylcarbonyls;
Z is a direct bond or a peptide linker composed of 2 to 30 arbitrary amino acid residues;
$X^1$ is a group selected from the group consisting of $-NR^b-R$, $-NR^b-COO-R$, $-NR^b-CO-R$, $-NR^b-CO-NR^c-R$, $-COO-R$, $-O-COO-R$, $-S-R$, $-CO-Y^a-S-R$, $-O-CO-Y^b-S-R$, $-NR^b-CO-Y^b-S-R$, and $-S-S-R$;
$R^a$, $R^b$ and $R^c$ are each independently a group selected from the group consisting of a hydrogen atom, $C_{1-20}$ alkyls, amino $C_{2-20}$ alkyls and hydroxy $C_{2-20}$ alkyls, and a group selected from the group consisting of -O- and $-NR^f-$ may be introduced in an alkyl moiety of $R^a$, $R^b$ or $R^c$, mentioned above;
$R^f$ is selected from the group consisting of a hydrogen atom, $C_{1-12}$ alkyls, amino $C_{2-12}$ alkyls and hydroxy $C_{2-12}$ alkyls, and a group selected from the group consisting of - O- and -NH- may be introduced in an alkyl moiety of $R^f$;
R is a steryl group;
Y is a $C_{2-30}$ alkylene or $-(CH_2CH_2O)_m-CH_2CH_2-$, and a group selected from the group consisting of -O-, $-NR^g-$ and -S-S- may be introduced in an alkylene moiety of Y;
$R^g$ is a group selected from the group consisting of a hydrogen atom, $C_{1-20}$ alkyls, amino $C_{2-20}$ alkyls and hydroxy $C_{2-20}$ alkyls, and a group selected from the group consisting of -O- and -NH- may be introduced in an alkyl moiety of $R^g$;
$Y^a$ is a $C_{1-5}$ alkylene;
$Y^b$ is a $C_{2-8}$ alkylene or a $C_{2-8}$ alkenylene; and
m is an integer of 1 or more and 100 or less],
and at least one repeating unit of general formula (II) shown below:

[Chemical Formula 2]

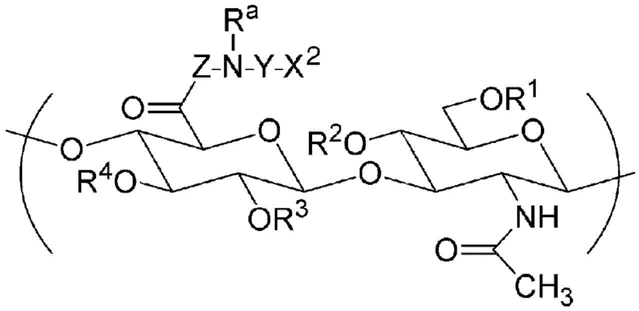

(II)

[in general formula (II), $R^1$, $R^2$, $R^3$, and $R^4$ are each independently a group selected from the group consisting of a hydrogen atom, $C_{1-6}$ alkyls, a formyl and $C_{1-6}$ alkylcarbonyls;
Z is a direct bond or a peptide linker composed of 2 to 30 arbitrary amino acid residues;
$X^2$ is a maleimide group;
$R^a$ is a group selected from the group consisting of a hydrogen atom, $C_{1-20}$ alkyls, amino $C_{2-20}$ alkyls and hydroxy $C_{2-20}$ alkyls, and a group selected from the group consisting of -O- and -$NR^f$- may be introduced in an alkyl moiety of $R^a$;
Y is a $C_{2-30}$ alkylene or -$(CH_2CH_2O)_m$-$CH_2CH_2$-, and a group selected from the group consisting of -O-, -$NR^g$- and -S-S- may be introduced in an alkylene moiety of Y;
$R^g$ is a group selected from the group consisting of a hydrogen atom, $C_{1-20}$ alkyls, amino $C_{2-20}$ alkyls and hydroxy $C_{2-20}$ alkyls, and a group selected from the group consisting of -O- and -NH- may be introduced in an alkyl moiety of $R^g$; and
m is an integer of 1 or more and 100 or less].

[7] The hyaluronic acid derivative according to any one of [1] to [6] mentioned above, wherein the steryl groups are cholesteryl groups.
[8] The hyaluronic acid derivative according to any one of [1] to [7] mentioned above, wherein an average particle diameter of the hyaluronic acid derivative when dissolved in a phosphate buffer solution (10 mM) at a concentration of 1 mg/mL is 250 nm or less, the average particle diameter being calculated by a dynamic light scattering method.
[9] A method for producing a cross-linked gel of a hyaluronic acid derivative, including reacting the hyaluronic acid derivative of any one of [1] to [8] mentioned above with a cross-linking agent having two or more cross-linkable groups to be gelated.
[10] The method for producing a cross-linked gel of a hyaluronic acid derivative according to [9] mentioned above, wherein the cross-linkable groups are thiol groups.
[11] A cross-linked gel of a hyaluronic acid derivative, wherein the cross-linked gel is a gel-like substance in which the hyaluronic acid derivative of any one of [1] to [8] mentioned above is chemically cross-linked with a cross-linking agent having two or more cross-linkable groups.
[12] The cross-linked gel of a hyaluronic acid derivative according to [11] mentioned above, wherein the cross-linkable groups are thiol groups.
[13] The cross-linked gel of a hyaluronic acid derivative according to [11] or [12] mentioned above, wherein a swelling ratio is 115% or less.
[14] A dried substance of a cross-linked gel of a hyaluronic acid derivative, wherein the dried substance is a dried substance of a gel-like substance in which the hyaluronic acid derivative of any one of [1] to [8] mentioned above is chemically cross-linked with a cross-linking agent having two or more cross-linkable groups.
[15] The dried substance of a cross-linked gel of a hyaluronic acid derivative according to [14] mentioned above, wherein the dried substance is a porous structure.
[16] A pharmaceutical composition containing the hyaluronic acid derivative of any one of [1] to [8] mentioned above.
[17] A pharmaceutical composition containing a cross-linked gel of a hyaluronic acid derivative, wherein the cross-linked gel is a gel-like substance in which the hyaluronic acid derivative of any one of [1] to [8] mentioned above is chemically cross-linked with a cross-linking agent having two or more cross-linkable groups.
[18] The pharmaceutical composition according to [17] mentioned above, further containing an active ingredient.

Advantageous Effects of Invention

The hyaluronic acid derivative according to the above-mentioned aspect makes it possible to provide a hyaluronic acid derivative which exhibits excellent swelling-suppression properties after gelation and a cross-linked gel of the hyaluronic acid derivative, as well as a pharmaceutical composition using the same.

BRIEF DESCRIPTION OF DRAWINGS

[FIG. 1A] FIG. 1A is an NMR chart of a cross-linked gel of a hyaluronic acid derivative (HA-C6-Chol-0%-Male) synthesized in Example 2.

[FIC. 1B] FIG. 1B is an NMR chart of a cross-linked gel of a hyaluronic acid derivative (HA-C6-Chol-1 %-Male) synthesized in Example 2.

[FIC. 1C] FIG. 1C is an NMR chart of a cross-linked gel of a hyaluronic acid derivative (HA-C6-Chol-5%-Male) synthesized in Example 2.

[FIG. 1D] FIG. 1D is an NMR chart of a cross-linked gel of a hyaluronic acid derivative (HA-C6-Chol-10%-Male) synthesized in Example 2.

[FIG. 1E] FIG. 1E is an NMR chart of a cross-linked gel of a hyaluronic acid derivative (HA-C6-Chol-15%-Male) synthesized in Example 2.

[FIG. 1F] FIG. 1F is an NMR chart of a cross-linked gel of a hyaluronic acid derivative (HA-C6-Chol-20%-Male) synthesized in Example 2.

[FIG. 1G] FIG. 1G is an NMR chart of a cross-linked gel of a hyaluronic acid derivative (HA-C6-Chol-30%-Male) synthesized in Example 2.

[FIG. 1H] FIG. 1H is an NMR chart of a cross-linked gel of a hyaluronic acid derivative (HA-C6-Chol-40%-Male) synthesized in Example 2.

[FIG. 2] FIG. 2 is a drawing that indicates changes in swelling ratio (%) over time from the start of gelation of cross-linked gels of HAMICHs with different introduction rates of cholesteryl groups in Example 2. Results of the cross-linked gels of HAMICHs prepared at a hyaluronic acid concentration of 7.0 mg/mL are indicated in FIG. 2(A), and results of the cross-linked gels of HAMICHs prepared at a hyaluronic acid concentration of 16.7 mg/mL are indicated in FIG. 2(B).

[FIG. 3] FIG. 3 is a drawing that indicates results of uptake ratios (%) of FITC-insulin in cross-linked gels of HAMICHs into which FITC-insulin was uptaken in Example 3.

[FIG. 4] FIG. 4 is a visible light photograph of external appearances of the cross-linked gels of HAMICHs in which FITC-insulin was uptaken in Example 3.

[FIG. 5] FIG. 5 indicates fluorescence microscopy images taken after freeze-dry treatment or freeze-thaw treatment of cross-linked gels of Cy5-HAMICH in which the introduction rate of cholesteryl groups was 19% (upper parts of FIG. 5) and cross-linked gels of Cy5-HAMICH in which the introduction rate of cholesteryl groups was 0% (lower parts of FIG. 5) in Example 4.

[FIG. 6] FIG. 6 is a drawing that indicates results of release ratio (%) of FITC-insulin, measured in Example 8, from cross-linked gels of HAMICHs in which FITC-insulin was uptaken.

[FIG. 7] FIG. 7 is a drawing that indicates the changes over time in swelling ratio (%) of cross-linked gels of HAMICHs from the start of gelation in Example 10. FIG. 7(A) indicates the results of the cross-linked gel of HAMICH (HA-C6-Chol-1%-Male 8.1%) of Test plot 9-1, and FIG. 7(B) indicates the results of the cross-linked gel of HAMICH (HA-C6-Chol-17%-Male 4.6%) of Test plot 9-1.

DESCRIPTION OF EMBODIMENTS

Although an embodiment according to the present invention (hereinafter, referred to as the "present embodiment") will be explained specifically below, the present invention is not limited to this, and various modifications may be carried out without departing from the gist of the present invention.

The terms used in the present specification will be explained, below.

The term "$C_{1-20}$ alkyl" used in the present specification means a straight-chain or branched-chain alkyl group having 1 to 20 carbon atoms, and examples thereof include: "$C_{1-4}$ alkyls" such as methyl, ethyl, n-propyl, iso-propyl, n-butyl, sec-butyl, iso-butyl, and tert-butyl; and further include n-pentyl, 3-methylbutyl, 2-methylbutyl, 1-methylbutyl, 1-ethylpropyl, n-hexyl, 4-methylpentyl, 3-methylpentyl, 2-methylpentyl, 1-methylpentyl, 3-ethylbutyl, and 2-ethylbutyl. Examples of the $C_{1-20}$ alkyl include $C_{1-12}$ alkyls having 1 to 12 carbon atoms, and $C_{1-6}$ alkyls having 1 to 6 carbon atoms.

The term "$C_{1-6}$ alkylcarbonyl" used in the present specification means an alkylcarbonyl in which the alkyl moiety is

the above-mentioned $C_{1-6}$ alkyl, and examples thereof include "$C_{1-4}$ alkylcarbonyls" such as acetyl, propionyl, n-propylcarbonyl, iso-propylcarbonyl, n-butylcarbonyl, sec-butylcarbonyl, iso-butylcarbonyl, and tert-butylcarbonyl.

The term "amino $C_{2-20}$ alkyl" used in the present specification means a straight-chain or branched-chain alkyl having 2 to 20 carbon atoms with an amino group as a substituent, in which the amino group may be located on a terminal carbon atom of the alkyl group, for example. Examples of the amino $C_{2-20}$ alkyl include amino $C_{2-12}$ alkyls having 2 to 12 carbon atoms.

The term "hydroxy $C_{2-20}$ alkyl" used in the present specification means a straight-chain or branched-chain alkyl group having 2 to 20 carbon atoms with a hydroxy group as a substituent, in which the hydroxy group may be located on a terminal carbon atom of the alkyl group, for example. Examples of the hydroxy $C_{2-20}$ alkyl include hydroxy$C_{2-12}$ alkyls having 2 to 12 carbon atoms.

The term "$C_{2-30}$ alkylene" used in the present specification means a straight-chain or branched-chain divalent saturated hydrocarbon group having 2 to 30 carbon atoms, and examples thereof include ethylene and propylene, and $C_{2-20}$ alkylenes having 2 to 20 carbon atoms, $C_{2-8}$ alkylenes having 2 to 8 carbon atoms, and groups of "$-(CH_2)_n-$" (wherein n is 2 or more and 30 or less, preferably 2 or more and 20 or less, and more preferably 2 or more and 15 or less).

The term "$C_{1-5}$ alkylene" used in the present specification means a straight-chain or branched-chain divalent saturated hydrocarbon group having 1 to 5 carbon atoms, and examples thereof include methylene, ethylene, and propylene.

The term "$C_{2-8}$ alkenylene" mentioned in the present specification means a straight-chain or branched-chain divalent saturated hydrocarbon group having 2 to 8 carbon atoms with at least one double bond, and examples thereof include -CH=CH-, - $C(CH_3)$=CH-, 2-butene-1,4-diyl, hepta-2,4-dien-1,6-diyl, and octa-2,4,6-triene-1,8-diyl. In a case where geometric isomers are present, each isomer and mixture thereof are also encompassed.

In the present specification, the term "swelling ratio" refers to the degree of increased volume of a cross-linked gel in which a cross-linked hyaluronic acid derivative is cross-linked under physiological salt concentration or in PBS(-) (in vivo) depending on the relationship between osmotic pressure and elastic pressure. Specifically, a cross-linked gel of a hyaluronic acid derivative in which the swelling ratio calculated by the following equation is a negative value is a gel in which the volume at the time of reaching equilibrium in PBS(-) decreases (shrinks) compared to the volume before gelation (which corresponds to the volume immediately after gelation). A cross-linked gel of a hyaluronic acid derivative in which the swelling ratio is a positive value is a gel in which the volume at the time of reaching equilibrium in PBS(-) increases (swells) compared to the volume immediately after gelation. In a cross-linked gel of a hyaluronic acid derivative, the smaller the absolute value of the swelling ratio, the smaller the change in volume of the cross-linked gel, which is preferable.

[Swelling ratio (%)] = ([volume ($mm^3$) of a composition containing a hyaluronic acid derivative at the time of reaching equilibrium after being gelated and then immersed in PBS(-)] / [volume ($mm^3$) of the composition containing a hyaluronic acid derivative before gelation]) $\times$100(%)

<<Hyaluronic acid derivative>>

A hyaluronic acid derivative according to the present embodiment has steryl groups introduced into at least a part of carboxyl groups in glucuronic acid moieties of the hyaluronic acid and maleimide groups introduced into at least a part of carboxyl groups in glucuronic acid moieties of the hyaluronic acid or hydroxyl groups in N-acetylglucosamine moieties thereof.

The reason why the swelling after gelation of the hyaluronic acid derivative according to the present embodiment is suppressed is not clear, the reason is assumed as follows. It is assumed that the decrease in the hydrophilicity of the entire molecule through the introduction of a combination of steryl groups and maleimide groups, which are hydrophobic groups, into carboxyl groups and hydroxyl groups, which are hydrophilic groups of a hyaluronic acid, contributes to the suppression of the swelling. In addition, it is assumed that the progress of the gelation in a relatively short time of 1 second to 30 minutes by particularly using a combination of steryl groups and maleimide groups as hydrophobic groups to be introduced into a hyaluronic acid also contributes to the suppression of the swelling. In particular, the swelling-suppression properties are poor in hyaluronic acid derivatives in which only maleimide groups are modified without modification of steryl groups, and therefore it is also assumed the elastic pressure generated by the strong hydrophobic interaction of steryl groups contributes to the swelling suppression effects. Hyaluronic acid derivatives modified with functional groups having strong interactions due to $\pi$-$\pi$ stacking, such as aromatic rings, may also have excellent swelling-suppression properties after gelation, in a similar manner to the hyaluronic acid derivative according to the present embodiment.

The swelling ratio of the hyaluronic acid derivative according to the present embodiment is preferably 115% or less, more preferably 110% or less, and even more preferably 105% or less. The swelling ratio of the hyaluronic acid derivative according to the present embodiment is preferably 85% or more, more preferably 90% or more, and even more preferably 95% or more. The swelling ratio of the hyaluronic acid derivative according to the present embodiment is particularly preferably 85% or more and 115% or less.

[Steryl groups]

The term "steryl group" used in the present specification is not particularly limited, as long as the term means a group having a steroid skeleton. Specific examples of steroid include cholesterol, cholestanol, campestanol, ergostanol, stigmastanol, coprostanol, stigmasterol, sitosterol, lanosterol, ergosterol, simiarenol, bile acid, testosterone, estradiol, progesterone, cortisol, cortisone, aldosterone, corticosterone, and deoxycortisterone. Examples of the steryl group include a cholesteryl group, a stigmasteryl group, a lanosteryl group, and an ergosteryl group, and among them, a cholesteryl group (particularly, a cholester-5-en-3β-yl group) is preferable.

In the hyaluronic acid derivative, the steryl group may be directly bonded to a hyaluronic acid or may be bonded thereto via a linker. All of the linkers that are contained in one molecule of the hyaluronic acid derivative and to be linked to steryl groups may be identical to each other, or may be different from each other in terms of the length or type thereof.

The term "linker" used herein refers to a group formed by linking a group that reacts with a carboxy group and a group that reacts with a steryl group via a chain group (spacer). Examples of the group that reacts with a carboxy group and the group that reacts with a steryl group include an amino group and a hydroxyl group. As the chain group, for example, a chain hydrocarbon group, a polyethylene glycol (PEG) chain, an arbitrary peptide linker that can be introduced by genetic engineering, or a synthesized compound linker may be used. The length of the linker is not particularly limited, and can be appropriately selected by those skilled in the art depending on the intended purpose. As the chain hydrocarbon group, $C_{1-30}$ alkylene is preferable, $C_{1-10}$ alkylene is more preferable, and $C_{1-6}$ alkylene is even more preferable. As the PEG group, a group in which the number of linked ethylene glycol groups is 1 to 15 is preferable, a group in which the number is 1 to 10 is more preferable, and a group in which the number is 1 to 5 is even more preferable. The peptide linker is 2 amino acids or more (the upper limit is not particularly limited, but usually 30 amino acids or less, preferably 20 amino acids or less), and particularly preferably 15 amino acids. In the hyaluronic acid derivative according to the present embodiment, at least a part of carboxyl groups in glucuronic acid moieties is preferably linked to steryl groups via linkers having a chain hydrocarbon group.

[Introduction rate of steryl groups]

The introduction rate of steryl groups in the hyaluronic acid derivative according to the present embodiment (hereinafter, may be simply referred to as "introduction rate of steryl groups") is preferably 0.5% or more, more preferably 1.0% or more, even more preferably 5.0% or more and still more preferably 10% or more. The introduction rate of steryl groups in the hyaluronic acid derivative according to the present embodiment is preferably 30% or less, more preferably 25% or less, even more preferably 20% or less, and still more preferably 15% or less. When the introduction rate of steryl groups is within the above-mentioned range, the swelling ratio can be sufficiently lowered, and a sufficient amount of drug can be uptaken when a cross-linked gel is formed.

The introduction rate of steryl groups can be measured by $^1$H-NMR measurement. That is, it can be calculated based on the following equation using the integral value of the peak derived from steryl groups of the hyaluronic acid derivative in $^1$H-NMR spectrum and the integral value of the peak ($COCH_3$, 1.6 ppm or more and 2.0 ppm or less, 3H) derived from acetyl groups of N-acetyl-D-glucosamine contained in the hyaluronic acid derivative. In the equation, $n_H$ indicates the number of hydrogen atoms corresponding to the peak. Specifically, for example, the measurement may be carried out in accordane with the method described later in Examples.

$$\text{[Introduction rate of steryl groups] (\%)} = \text{(Integrated value of peak derived from steryl groups]} \times 3/n_H) / \text{[Integrated value of peak derived from acetyl groups of N-acetyl-D-glucosamine)} \times 100$$

[Maleimide group]

In the hyaluronic acid derivative, maleimide groups (N-maleimide groups) may be directly bonded to hyaluronic acid or may be bonded thereto via linkers. The linkers that are contained in one molecule of the hyaluronic acid derivative and to be linked to maleimide groups may be identical to each other or may be different from each other in terms of the length or type thereof.

The term "linker" used herein refers to a group formed by linking a group that reacts with a carboxy group and a group that reacts with an NH group in a maleimide group via a chain group. Examples of the group that reacts with a carboxy group include an amino group and a hydroxyl group. Examples of the group that reacts with an NH group in a maleimide group include a carboxy group and a hydroxyl group. Examples of the chain group include the same groups as

those mentioned as the chain group in the linker that links the hyaluronic acid derivative and steryl group. It is preferable in the hyaluronic acid derivative according to the present embodiment that at least a part of carboxyl groups in glucuronic acid moieties and hydroxyl groups in N-acetylglucosamine moieties be linked with NH groups of maleimide groups via linkers each having a chain hydrocarbon group.

[Introduction rate of maleimide groups]

The introduction rate of maleimide groups in the hyaluronic acid derivative according to the present embodiment (hereinafter, may be simply referred to as "introduction rate of maleimide groups") is preferably 1.0% or more, more preferably 3.0% or more, even more preferably 4.5% or more, still more preferably 5.0% or more, still more preferably 8.0% or more, and particularly preferably 10% or more. In the hyaluronic acid derivative according to the present embodiment, the introduction rate of maleimide groups is preferably 25% or less, and more preferably 20% or less. Among them, the introduction rate of maleimide groups in the hyaluronic acid derivative according to the present embodiment makes it possible to sufficiently decrease the swelling ratio when the introduction rate of steryl groups is within the above-mentioned range.

The introduction rate of maleimide groups may be measured by $^1$H-NMR measurement. The $^1$H-NMR spectrum is obtained by measuring a solution in which the hyaluronic acid derivative according to the present embodiment is dissolved in a heavy solvent of a 0.02 N DCl DMSO-d6 / D2O mixture liquid (2 N DCl D2O: DMSO-d6 = 1:99). The introduction rates of cholesteryl groups and maleimide groups relative to hyaluronic acid units are calculated from the integral value of the peak ($COCH_3$, 1.6 to 2.0 ppm; 3H) derived from acetyl groups in glucosamine moieties of the hyaluronic acid derivative in the $^1$H-NMR spectrum, the integral value of the peak ($CH_3$, 0.7 ppm; 3H) derived from methyl groups in cholesteryl groups, and the integral value of the peak (-CH=CH-, 6.9 ppm; 2H) derived from maleimide groups, in accordance with the equation shown below. Since the peak derived from cholesteryl groups (5H) overlaps with the peak around 1.6 to 2.0 ppm including the peak derived from acetyl groups of glucosamine moieties, the value calculated by subtracting 5/3 of the integral value of the peak (0.7 ppm) derived from methyls in cholesteryl groups from the integral value of the peak around 1.6 to 2.0 ppm (namely, ([integral value of peak (1.6 to 2.0 ppm)] - [integral value of peak (0.7 ppm)] × 5/3) was used as the integral value (corrected value) of the acetyl groups derived from hyaluronic acid to calculate the introduction rate. First, the introduction rate (%) of cholesteryl groups is calculated in accordance with the following equation.

[Introduction rate (%) of cholesteryl groups]

= [integral value of peak (0.7 ppm) derived from cholesteryl groups] / [integral value of peak (1.6 to 2.0 ppm, corrected value) derived from acetyl groups in N-acetyl-D-glucosamine moieties] × 100(%)

In addition, the introduction rate (%) of maleimide groups may be calculated from the integral value of the peak derived from maleimide groups and the integral value of the peak ($COCH_3$, 1.6 ppm or more and 2.0 ppm or less, 3H) derived from cholesteryl groups, in accordance with the following equation. Specifically, measurement can be carried out in accordance with the method described in Examples mentioned below.

[Introduction rate (%) of maleimide groups]

= ([integral value of peak derived from maleimide groups] × 3) / ([integral value of peak derived from cholesteryl groups] × 2) × [introduction rate (%) of cholesteryl groups]

The total of the introduction rate of steryl groups and the introduction rate of maleimide groups in the hyaluronic acid derivative according to the present embodiment is preferably 55% or less, more preferably 45% or less, even more preferably less than 45%, still more preferably 40% or less, and particularly preferably 35% or less. The total of the introduction rate of steryl groups and the introduction rate of maleimide groups in the hyaluronic acid derivative according

to the present embodiment is preferably 1.0% or more, more preferably 2.5% or more, and even more preferably 5.0% or more. When the total of the introduction rate of steryl groups and the introduction rate of maleimide groups in the hyaluronic acid derivative is within the above-mentioned range, the change in volume after gelation under physiological salt concentration (in vivo) can be further decreased, and, when used as a DDS carrier, the cross-linking density can be increased, thereby making it possible to extend the sustained release period of a drug.

Specifically, preferable examples of the hyaluronic acid derivative include a hyaluronic acid derivative having at least one repeating unit of the following general formula (I) (hereinafter, may be referred to as "repeating unit (I)") and at least one repeating unit of the following general formula (II) (hereinafter, may be referred to as "repeating unit (II)").

[Chemical Formula 3]

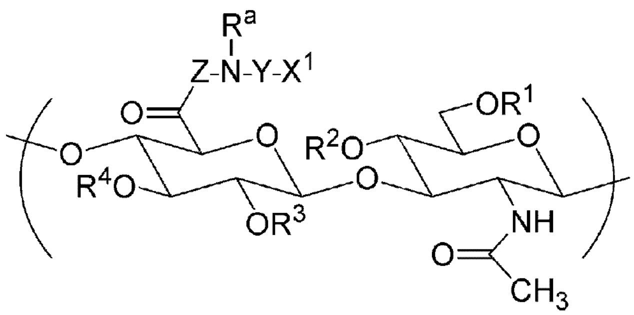

(I)

(In the general formula (I), $R^1$, $R^2$, $R^3$, and $R^4$ are each independently selected from the group consisting of a hydrogen atom, $C_{1-6}$ alkyls, a formyl and $C_{1-6}$ alkylcarbonyls;

Z is a direct bond or a peptide linker composed of 2 to 30 arbitrary amino acid residues;

$X^1$ is a group selected from the group consisting of groups of the following formulae:

$-NR^b-R$,

$-NR^b-COO-R$,

$-NR^b-CO-R$,

$-NR^b-CO-NR^c-R$,

$-COO-R$,

$-O-COO-R$,

$-S-R$,

$-CO-Y^a-S-R$,

$-O-CO-Y^b-S-R$,

$-NR^b-CO-Y^b-S-R$,

and

$-S-S-R$;

$R^a$, $R^b$ and $R^c$ are each independently selected from the group consisting of a hydrogen atom, $C_{1-20}$ alkyls, amino $C_{2-20}$ alkyls and hydroxy $C_{2-20}$ alkyls, and a group selected from the group consisting of -O- and $-NR^f-$ may be introduced in an alkyl moiety thereof;

$R^f$ is selected from the group consisting of a hydrogen atom, C1-12 alkyls, amino C2-12 alkyls and hydroxy C2-12 alkyls, and a group selected from the group consisting of -O- and -NH- may be introduced in an alkyl moiety thereof;

R is a steryl group;

Y is a C2-30 alkylene or $-(CH_2CH_2O)_m-CH_2CH_2-$, and a group selected from the group consisting of -O-, $-NR^g-$ and -S-S- may be introduced in an alkylene moiety thereof;
$R^g$ is selected from the group consisting of a hydrogen atom, $C_{1-20}$ alkyls, amino $C_{2-20}$ alkyls and hydroxy $C_{2-20}$ alkyls, and a group selected from the group consisting of - O- and -NH- may be introduced in an alkyl moiety thereof;
$Y^a$ is a $C_{1-5}$ alkylene;
$Y^b$ is a $C_{2-8}$ alkylene or a $C_{2-8}$ alkenylene; and
m is an integer of 1 or more and 100 or less.)

[Chemical Formula 4]

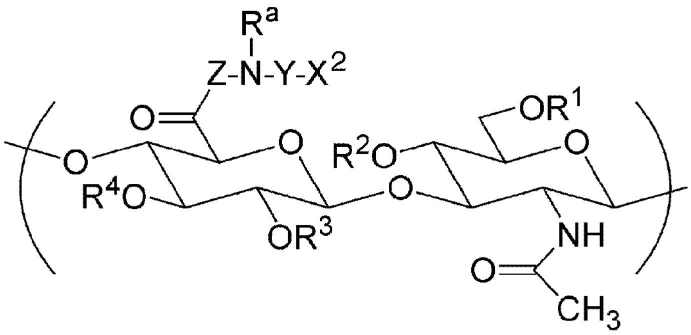

(II)

(In general formula (II), $R^1$, $R^2$, $R^3$, and $R^4$ are each independently selected from the group consisting of a hydrogen atom, $C_{1-6}$ alkyls, a formyl and $C_{1-6}$ alkylcarbonyls;
Z is a direct bond or a peptide linker composed of 2 to 30 arbitrary amino acid residues;
$R^a$ is selected from the group consisting of a hydrogen atom, $C_{1-20}$ alkyls, amino $C_{2-20}$ alkyls and hydroxy $C_{2-20}$ alkyls, and a group selected from the group consisting of - O- and $-NR^f-$ may be introduced in an alkyl moiety thereof;
$R^f$ is selected from the group consisting of a hydrogen atom, $C_{1-12}$ alkyls, amino $C_{2-12}$ alkyls and hydroxy $C_{2-12}$ alkyls, and a group selected from the group consisting of - O- and -NH- may be introduced in an alkyl moiety thereof;
$X^2$ is a maleimide group;
Y is a $C_{2-30}$ alkylene or $-(CH_2CH_2O)_m-CH_2CH_2-$, and a group selected from the group consisting of -O-, $-NR^g-$ and -S-S- may be introduced in an alkylene moiety thereof;
$R^g$ is selected from the group consisting of a hydrogen atom, $C_{1-20}$ alkyls, amino $C_{2-20}$ alkyls and hydroxy $C_{2-20}$ alkyls, and a group selected from the group consisting of - O- and -NH- may be introduced in an alkyl moiety thereof; and
m is an integer of 1 or more and 100 or less.)

[Repeating unit (I)]

The group "$-Z-N(R^a)-Y-X^1$" in the general formula (I) includes a group selected from the group consisting of groups of the following formulae:

$-NH-(CH_2)_{mz}-NH-R;$

$-NH-(CH_2)_{mz}-NH-COO-R;$

$-NH-(CH_2CH_2O)_m-CH_2CH_2-NH-COO-R;$

$-NH-(CH_2)_{mz}-COO-R;$

$-NH-(CH_2CH_2O)_m-CH_2CH_2-COO-R;$

$-NH-(CH_2)_{mz}-O-COO-R;$

$-NH-(CH_2CH_2O)_m-CH_2CH_2-O-COO-R;$

$-NH-(CH_2)_{mz}-S-R;$

$-NH-(CH_2CH_2O)_m-CH_2CH_2-S-R;$

$-NH-(CH_2)_{mz}-O-CO-CH(R^8)-CH_2-S-R;$

$-NH-(CH_2)_{mz}-NHCO-CH(R^8)-CH_2-S-R;$

$-NH-(CH_2CH_2O)_m-CH_2CH_2-NHCO-CH(R^8)-CH_2-S-R;$

$-NH-(CH_2CH_2O)_m-CH_2CH_2-O-CO-CH(R^8)-CH_2-S-R;$

$-NH-(CH_2)_{mz}-S-S-R;$

and

$-Z-NR^a-Y-NR^b-COO-R.$

(Here, mz is an integer of 2 or more and 30 or less, $R^8$ is a hydrogen atom or a methyl group, and R and m are as already defined in the present specification.)

This group is preferably a group selected from

$-NH-(CH_2)_{mz}-NH-COO-R;$

$-NH-(CH_2CH_2O)_m-CH_2CH_2-NH-COO-R;$

and

$-NH-(CH_2)_{mz}-S-S-R.$

(Here, mz, R and m are as already defined in the present specification.)

(Z)

In the general formula (I), Z is preferably a direct bond. In another aspect, when Z is a peptide linker, $X^1$ is preferably $-NR^b-COO-R$. In another aspect, Z may be a peptide linker of $-NH-[CH(-Z^a)-CONH]_{n-1}-CH(-Z^a)-CO-$, wherein n is an integer of 2 or more and 30 or less, and $Z^a$ is each independently a substituent in an α-amino acid of $H_2N-CH(-Z^a)-COOH$. The peptide linker is bonded to a carboxy group of the glucuronic acid moiety at the N-terminal and bonded to a group of $-N(-R^a)-Y-X^1$ at the C-terminal. Examples of the amino acid that can be used as the amino acid residue of the peptide linker include α-amino acids such as natural (L-type) amino acids such as alanine, arginine, asparagine (Asn), aspartic acid, cysteine, glutamine, glutamic acid, glycine (Gly), histidine, isoleucine, leucine (Leu), lysine, methionine, phenylalanine (Phe), proline, serine, threonine, tryptophan, tyrosine, and valine, and D-type thereof, and all α-amino acids, including synthesized amino acids, can be used. Namely, examples of $Z^a$ include-$CH_3$, $H_2NC(NH)NH(CH_2)_3$-, $H_2NCOCH_2$-and the like. In addition, n Z may be identical to or different from each other. n is an integer of 2 or more and 30 or less, preferably 2 or more and 10 or less, and more preferably 2 or more and 4 or less. Preferred examples of the peptide linker include -Gly-Phe-Leu-Gly-, -Asn-Phe-Phe-, - Phe-Phe-, and -Phe-Gly-.

(Y)

In the general formula (I), Y is preferably selected from the group consisting of - $(CH_2)_{n1}$-and-$(CH_2CH_2O)_{m1}-CH_2CH_2$- (wherein n1 is an integer of 2 or more and 20 or less, preferably an integer of 2 or more and 15 or less, more preferably an integer of 2 or more and 12 or less, and even more preferably an integer of 2 or more and 6 or less, and m1 is an integer of 1 or more and 4 or less). Specifically, -$(CH_2)_2$-, -$(CH_2)_6$-, -$(CH_2)_8$-, - $(CH_2)_{12}$-, or -$(CH_2CH_2O)_2-CH_2CH_2$-is preferable. Furthermore, Y is preferably a group selected from the group consisting of-$(CH_2)_2$-, -$(CH_2)_6$-, -$(CH_2)_8$- and -$(CH_2)_{12}$-, and more preferably -$(CH_2)_6$-, from the viewpoint of exhibiting high precipitation forming ability at physiological salt concentration while achieving high solubility in pure water or at a low salt concentration.

Y may also be, for example, $-CH_2CH_2O-CH_2CH_2-S-S-CH_2CH_2O-CH_2CH_2-$, - $(CH_2CH_2O)_2-CH_2CH_2-S-S-CH_2CH_2O-CH_2CH_2-$, $-CH_2CH_2O-CH_2CH_2-S-S-(CH_2CH_2O)_2-CH_2CH_2-$, $-(CH_2CH_2O)_2-CH_2CH_2-S-S-(CH_2CH_2O)_2-CH_2CH_2-$ or the like.

($Y^a$)

$Y^a$ is preferably -$CH_2$-or-$CH_2-CH_2$-.

$(Y^b)$

$Y^b$ is preferably-$CH_2$-$CH_2$-, -$CH(CH_3)CH_2$-, 2-butene-1,4-diyl, hepta-2,4-diene-1,6-diyl or octa-2,4,6-triene-1,8-diene, and more preferably-$CH_2$-$CH_2$-or-$CH(CH_3)CH_2$-.

Examples of the group of "-Z-N ($R^a$)Y-$X^1$" include -NH-$(CH_2)_2$-NH-CO-cholesteryl, -NH-$(CH_2)_4$-NH-$(CH_2)_3$-NH-$(CH_2)_3$-NH-COO-cholesteryl, -NH-$(CH_2)_3$-NH-$(CH_2)_4$-NH-$(CH_2)_3$-NH-COO-cholesteryl, -NH-$(CH_2)_4$-NH-$(CH_2)_3$-NH-COO-cholesteryl, -NH-$(CH_2)_4$-N(-$(CH_2)_3$-$NH_2$)-COO-cholesteryl, -NH-$(CH_2)_3$-NH-$(CH_2)_4$-N(-$(CH_2)_3$-$NH_2$)-COO-cholesteryl, -NH-$(CH_2)_3$-NH-$(CH_2)_4$-N(-$(CH_2)_3$-NH-$(CH_2)_3$-$NH_2$)-COO-cholesteryl, -NH-$(CH_2)_3$-NH-$(CH_2)_4$-N(-$(CH_2)_3$-$NH_2$)-CO-NH-cholesteryl, -NH-$(CH_2)_3$-NH-$(CH_2)_4$-N(-$(CH_2)_3$-$NH_2$)-CO-cholesteryl, and -NH-$(CH_2)_3$-NH-$(CH_2)_4$-N(-$(CH_2)_3$-$NH_2$)-cholesteryl. Preferable examples thereof include a group of "-Z-N($R^a$)Y-$X^1$", in which $R^a$, $R^b$ and $R^c$ are hydrogen atoms, Y is a linear $C_{2-30}$ alkylene or -$(CH_2CH_2O)_m$-$CH_2CH_2$-, $Y^a$ is a linear $C_{1-5}$ alkylene, and $Y^b$ is a linear $C_{2-8}$ alkylene or a linear $C_{2-8}$ alkenylene.

In particular, as the repeating unit (I), a repeating unit of the general formula (I) in which Z is a direct bond, $R^a$ is a hydrogen atom or a $C_{1-6}$ alkyl, Y is a $C_{2-30}$ alkylene or -$(CH_2CH_2O)_m$-$CH_2CH_2$-, $X^1$ is a group of -$NR^{b1}$-COO-R, $R^{b1}$ is a hydrogen atom or a $C_{1-6}$ alkyl, and R is a steryl group is preferable; a repeating unit of the general formula (I) in which Z is a direct bond, $R^a$ is a hydrogen atom, Y is a $C_{2-30}$ alkylene or -$(CH_2CH_2O)_m$-$CH_2CH_2$-, $X^1$ is a group of -$NR^{b1}$-COO-R, $R^{b1}$ is a hydrogen atom or a $C_{1-6}$ alkyl, and R is a steryl group is more preferable; a repeating unit of the general formula (I) in which Z is a direct bond, $R^a$ is a hydrogen atom, Y is a $C_{2-30}$ alkylene or -$(CH_2CH_2O)_m$-$CH_2CH_2$-, $X^1$ is a group of -$NR^{b1}$-COO-R, $R^{b1}$ is a hydrogen atom or a $C_{1-6}$ alkyl, and R is a cholesteryl group is even more preferable; a repeating unit of the general formula (I) in which Z is a direct bond, $R^a$ is a hydrogen atom, -Y-$X^1$- is -$(CH_2)_2$-NH-COO-cholesteryl, -$(CH_2)_4$-NH-COO-cholesteryl, -$(CH_2)_5$-NH-COO-cholesteryl, -$(CH_2)_6$-NH-COO-cholesteryl, -$(CH_2)_{12}$-NH-COO-cholesteryl, or $(CH_2CH_2O)_2$-$CH_2CH_2$-NH-COO-cholesteryl is still more preferable; and a repeating unit of the general formula (I) in which Z is a direct bond, $R^a$ is a hydrogen atom, -Y-$X^1$- is -$(CH_2)_2$-NH-COO-cholesteryl, -$(CH_2)_4$-NH-COO-cholesteryl, -$(CH_2)_5$-NH-COO-cholesteryl, or -$(CH_2)_6$-NH-COO-cholesteryl is particularly preferable.

As the repeating unit (II), a repeating unit of the general formula (II) in which Z is a direct bond, $R^a$ is a hydrogen atom or a $C_{1-6}$ alkyl, Y is a $C_{2-30}$ alkylene or - $(CH_2CH_2O)_m$-$CH_2CH_2$-, and $X^2$ is a maleimide group is preferable; a repeating unit of the general formula (II) in which Z is a direct bond, $R^a$ is a hydrogen atom or a $C_{1-6}$ alkyl, Y is a $C_{2-30}$ alkylene, and $X^2$ is a maleimide group is more preferable; a repeating unit of the general formula (II) in which Z is a direct bond, $R^a$ is a hydrogen atom, Y is a $C_{2-30}$ alkylene, and $X^2$ is a maleimide group is even more preferable; and a repeating unit of the general formula (II) in which Z is a direct bond, $R^a$ is a hydrogen atom, Y is a $C_{2-10}$ alkylene, and $X^2$ is a maleimide group is still more preferable

In the hyaluronic acid derivative according to the present embodiment, two or more repeating units (I) contained in one molecule of the hyaluronic acid derivative may be entirely the same repeating unit or two or more different types of repeating unit from each other.

In the hyal) contained in one molecule of the hyaluronic acid derivative may be entirely the same repeating unit or maluronic acid derivative according to the present embodiment, two or more repeating units (Iy be composed of two or more different types of repeating unit from each other.

The proportion of the total unit number of the repeating unit (I) and the repeating unit (II) relative to the total unit number of repeating units in the hyaluronic acid derivative according to the present embodiment is preferably 40% or less, more preferably 35% or less, and even more preferably 30% or less. The proportion of the total unit number of the repeating unit (I) and the repeating unit (II) relative to the total unit number of repeating units in the hyaluronic acid derivative according to the present embodiment is preferably 1.0% or more, more preferably 2.5% or more, and even more preferably 5.0% or more. When the total unit number of the repeating unit (I) and the repeating unit (II) in the hyaluronic acid derivative is within the above-mentioned range, the change in volume after gelation can be further decreased, and, when used as a DDS carrier, the uptake ratio of a drug can be increased.

[Weight-average molecular weight Mw]

The weight-average molecular weight of the hyaluronic acid derivative according to the present embodiment is not particularly limited, and is preferably, for example, 1,000 (1 k) or more and 1,000,000 (1,000 k) or less, more preferably 5 k or more and 300 k or less, even more preferably 6 k or more and 200 k or less, and particularly preferably 30 k or more and 130 k or less.

The weight-average molecular weight of the hyaluronic acid derivative according to the present embodiment is preferably 250 kDa or less, more preferably 200 kDa or less, and even more preferably 150 kDa or less from the viewpoint that it becomes easy to further sufficiently decrease the change in volume after gelation and to control the particle size of a cross-linked gel of hyaluronic acid, obtained by cross-linking, within an appropriate range. In addition, the weight-average molecular weight is preferably 4 kDa or more, more preferably 20 kDa or more, even more preferably 30 kDa or more, and still more preferably 40 kDa or more, from the viewpoint of gelation of the hyaluronic acid derivative. In particular, the

weight-average molecular weight of the hyaluronic acid derivative according to the present embodiment is preferably 50 kDa or more and more preferably 90 kDa or more. The molecular weight of the hyaluronic acid derivative can be generally controlled using raw materials having corresponding molecular weights.

The weight-average molecular weight Mw and the number-average molecular weight Mn of the hyaluronic acid derivative can be generally controlled using raw materials having each corresponding molecular weight.

The weight-average molecular weight Mw and the number-average molecular weight Mn of the hyaluronic acid derivative can be measured by a size exclusion chromatography multi-angle light scattering detector (SEC-MALS), for example. The weight-average molecular weight Mw and the number-average molecular weight Mn of the hyaluronic acid derivative can be measured by the method described specifically in Examples mentioned below.

[Average particle diameter (nm) of nanoparticles]

The hyaluronic acid derivative according to the present embodiment forms nanoparticles in an aqueous solution. Although the size of the nanoparticles formed by the hyaluronic acid derivative according to the present embodiment is not particularly limited, the average particle diameter is preferably, for example, 500 nm or less, more preferably 450 nm or less, even more preferably 400 nm or less, still more preferably 300 nm or less, and particularly preferably 250 nm or less. The average particle diameter of nanoparticles formed by the hyaluronic acid derivative according to the present embodiment is preferably 10 nm or more, more preferably 20 nm or more, and even more preferably 50 nm or more.

In the present specification, the "average particle diameter of nanoparticles of the hyaluronic acid derivative" is a z-average particle diameter measured by a dynamic light scattering method. The measurement of the z-average particle diameter by the dynamic light scattering method may be carried out, for example, using a solution in which a hyaluronic acid derivative is dissolved in a phosphate buffer (10 mM) at a concentration of 1 mg/mL.

The nanoparticles formed by the hyaluronic acid derivative according to the present embodiment are hollow, and can encapsulate various substances inside. For example, nanoparticles with a target substance encapsulated inside and an outer shell made of the hyaluronic acid derivative are formed by dissolving the target substance to be encapsulated together with the hyaluronic acid derivative in an aqueous solution. The nanoparticles of the hyaluronic acid derivative according to the present embodiment can favorably encapsulate relatively large substances or substances that are poorly soluble in water. From these points of view, the hyaluronic acid derivative according to the present embodiment is suitable as a base material of the drug delivery system, and is particularly preferable as a base material of the sustained-release drug delivery system.

A cross-linked gel can be obtained by reacting the hyaluronic acid derivative according to the present embodiment with a cross-linking agent while encapsulating a drug without denaturing the drug.

[Method for producing hyaluronic acid derivative]

The hyaluronic acid derivative according to the present embodiment can be obtained by, for example, converting carboxyl groups of glucuronic acids to amides, introducing steryl groups into at least a part thereof directly or via linkers, and introducing maleimide groups into another part thereof directly or via linkers. The introduction rate of steryl groups can be controlled by adjusting the amount of a compound having steryl groups that react with a raw material hyaluronic acid or a derivative thereof, and the introduction rate of maleimide groups can be controlled by adjusting the amount of a compound having maleimide groups that react with the raw material hyaluronic acid or the derivative thereof. The compound having steryl groups may be reacted with the raw material hyaluronic acid, and the compound having maleimide groups may be reacted with the obtained reaction product. The compound having maleimide groups may be reacted with the raw material hyaluronic acid, and the compound having steryl groups may be reacted with the obtained reaction product. The compound having maleimide groups and the compound having steryl groups may be added to the raw material hyaluronic acid and reacted together in the reaction system. Methods for introducing maleimide groups or steryl groups into a hyaluronic acid as a raw material can be carried out by appropriately modifying the methods described in, for example, Japanese Unexamined Patent Application Publication No. 2021-123597, Japanese Unexamined Patent Application Publication No. 2022-013861, Japanese Unexamined Patent Application Publication No. 2022-044579., or the like.

Specific examples of the method for converting carboxy groups of glucuronic acids to amides and introducing steryl groups or maleimide groups thereinto include a method in which a raw material hyaluronic acid or a derivative thereof is ion-exchanged to a tetraalkylammonium salt (such as tetrabutylammonium (TBA) salt), and the hyaluronate is reacted with amines having introduced steryl groups (particularly cholesteryl groups) in a solvent in the presence of an appropriate condensing agent.

The condensing agent available in the above-mentioned reaction is not particularly limited, and examples thereof include 4-(4,6-dimethoxy-1,3,5-triazine)-4-methylmorpholinium (DMT-MM), N,N'-carbonyldiimidazole (CDI), N,N'-dicyclohexylcarbodiimide (DCC), N-ethoxycarbonyl-2-ethoxy-1,2-dihydroquinoline (EEDQ), 2-benzotriazole-1,1,3,3-tetramethyluronium tetrafluoroborate (TBTU), 3,4-dihydro-3-hydroxy-4-oxo-1,2,3-benzotriazine (HODhbt), benzotriazole-1-oxy-tris-pyrrolidino-phosphonium hexafluorophosphate (PyBOP), benzotriazole-1-yl-oxy-tris(dimethylamino)phosphonium hexafluorophosphate (BOP), 1-ethyl-3-(3-dimethylaminopropyl)carbodiimide (EDC), and N-hydroxysuccinimide (NHS).

Although there is no particular limitation, DMT-MM is preferable in terms that the reaction proceeds efficiently even in a mixed solvent of water and organic solvent. **In** addition, the use of DMT-MM as a condensing agent makes it possible to highly selectively form amide bonds between amino groups and carboxy groups in a system in which many hydroxy groups coexist, while suppressing the formation of ester bonds. The use of this condensing agent makes it possible to prevent, for example, the reaction of an alcohol solvent with carboxy groups in hyaluronic acid moieties, or the formation of undesired crosslinks caused by intramolecular or intermolecular bonds between carboxy groups and hydroxy groups that are simultaneously present in the hyaluronic acid moieties.

Examples of the solvent used in the reaction in which steryl groups are introduced include water, DMSO, methanol, ethanol, propanol, butanol, isopropanol, polyhydric alcohols, acetonitrile, DMF, THF, dichloromethane, chloroform, hexane, diethyl ether, ethyl acetate, and mixed solvents thereof. The polyhydric alcohols may be divalent alcohols or trivalent alcohols. Examples of the divalent alcohols include ethylene glycol, diethylene glycol, propylene glycol, dipropylene glycol, neopentyl glycol, 1,4-butanediol, and 1,6-hexanediol. Examples of the trivalent alcohols include glycerin and trimethylolpropane.

In the reaction in which steryl groups are introduced, the pH of the reaction system is preferably under acidic conditions. Succinimide groups can be generated by inactivation of maleimide groups under basic conditions.

Alternatively, a raw material hyaluronic acid or a derivative thereof may be ion-exchanged to a tetraalkylammonium salt (such as tetrabutylammonium (TBA) salt), and the hyaluronate may be reacted with spacer moieties in a solvent in the presence of an appropriate condensing agent (at this time, protection and deprotection reactions may be carried out as necessary) to convert carboxy groups (-COOH) of the raw material hyaluronic acid or the derivative thereof, followed by reacting the resultant with an appropriate reagent. Examples of combinations of groups derived from carboxy groups and reaction reagents are shown below.

$-CONR^a-Y-NR^bH$ + Hal-R;

$-CONR^a-Y-NR^bH$ + Hal-COOR;

$-CONR^a-Y-NR^bH$ + HOCO-R;

$-CONR^a-Y-NR^bH$ + Hal-CO-R;

$-CONR^a-Y-NR^b-COOH$ + $HNR^c-R$;

$-CONR^a-Y-NR^b-CO-NR^cH$ + Hal-R;

$-CONR^a-Y-NR^bH$ + $HOCO-NR^c-R$;

$-CONR^a-Y-NR^bH$ + $Hal-CO-NR^c-R$;

$-CONR^a-Y-COOH$ + HO-R

$-CONR^a-$ Y-OH + Hal-CO-R;

$-CONR^a-Y-OCOOH$ + HO-R;

$-CONR^a-Y-OCOOH$ + Hal- R;

$-CONR^a-Y-OCO-Hal$ + HO-R;

$-CONR^a-Y-SH$+ Hal-R;

$-CONR^a-Y-Hal$ + HS-R;

$-CONR^a-Y-CO-Y^a-Hal$ + HS-R;

$-CONR^a-Y-CO-Y^a-SH$ + Hal-R;

$-CONR^a-Y-O-CO-CH=CH_2 + HS-R;$

$-CONR^a-Y-NR^b-CO-CH(CH_3)=CH_2 + HS-R;$

$-CONR^a-Y-SH + HS-R;$

$-COZ-OH + HNRa-Y-NR^b-COO-R;$

and

$-COZ-NR^a-Y-NR^bH + Hal-COO-R.$

(In the formulae, $R^a$, $R^b$, $R^c$, Y, $Y^a$, $Y^b$, and Z have been already defined in the present specification, and Hal is a halogen atom selected from the group consisting of fluorine atom, a chlorine atom, a bromine atom, and an iodine atom).

Examples of the reaction patterns include a dehydrohalogenation reaction, a condensation reaction, a dehydration reaction, a nucleophilic addition reaction such as Michael addition, and an oxidative disulfide formation reaction. These are well-known reactions and may be carried out by those skilled in the art by appropriately selecting and finding favorable reaction conditions. When the converted product or the reaction product has a carboxyl group, the converted product or the reaction product may be made to be an N-hydroxysuccinimide (hereinafter, also referred to as "NHS") ester and then reacted.

Additional examples thereof include a method in which 2-aminoethyl 2-pyridyl disulfide is reacted with a carboxyl group of a raw material hyaluronic acid or a derivative thereof to prepare a hyaluronic acid derivative into which a spacer having a mercapto group modified with a leaving group at the terminal is introduced, followed by subjecting the hyaluronic acid derivative to a nucleophilic substitution reaction with thiocholesterol to form a disulfide bond.

Additional examples thereof include a method in which a hyaluronic acid or derivative thereof in which some of spacers are introduced into carboxyl groups thereof and a hyaluronic acid or derivative thereof in which some of the spacers are introduced into steryl groups thereof are prepared, followed by reacting them. Although some of specific examples have been described above, when -S-S- is introduced into Y, there is also a method in which a hyaluronic acid derivative in which a spacer having a mercapto group at the terminal is introduced into a carboxy group of the hyaluronic acid and a steryl group in which a spacer having a mercapto group at the terminal is introduced are prepared, followed by reacting them oxidatively to form a disulfide bond. At this time, one of the mercapto groups may be reacted with 2-mercaptopyridine to form a disulfide, and then substituted with the other of the mercapto groups.

Furthermore, after the hyaluronic acid derivative is prepared, another substituent may be further introduced. For example, 0.1% or more and 95.0% or less, and preferably 10% or more and 60% or less of carboxyl groups in the hyaluronic acid derivative may be converted to $-CO-X^z$ [wherein, $X^z$ is a group selected from the group consisting of the following groups:

$-NH-(CH_2)_{p1}-O-CO-C(R^{17})=CH_2;$

$-NH-(CH_2)_{p1}-O-CO-CH(R^{17})-CH_2-S-CH_2-CH(OH)-CH(OH)-CH_2-SH;$

$-NH-(CH_2)_{p1}-SH;$

$-NH-(CH_2)_{p1}-NH-CO-C(R^{17})=CH_2;$

$-NH-(CH_2)_{p1}-NH-C(=NH)-(CH_2)_3-SH;$

$-NH-(CH_2)_{p1}-NH-CO-(CH_2)_r-SH;$

$-NH-(CH_2)_{p1}-NH-CO-CH(R^{17})-CH_2-S-CH_2-CH(OH)-CH(OH)-CH_2-SH;$

$-NH-(CH_2)_{p1}-NH-CO-CH(NH_2)-CH_2-SH;$

$-NH-(CH_2)_{p1}-NH-CO-CH(NH_2)-(CH_2)_2-SH;$

$-NH-NH-CO-(CH_2)_4-CO-NH-NH-C(=NH)-(CH_2)_3-SH;$

$-NH-(CH_2-CH_2-O)_q-CH_2-CH_2-O-CO-C(R^{17})=CH_2$;

$-NH-(CH_2-CH_2-O)_q-CH_2-CH_2-O-CO-CH(R^{17})-CH_2-S-CH_2-CH(OH)-CH(OH)-CH_2-SH$;

$-NH-(CH_2-CH_2-O)_q-CH_2-CH_2-SH$;

$-NH-(CH_2-CH_2-O)_q-CH_2-CH_2-NH-CO-C(R^{17})=CH_2$;

$-NH-(CH_2-CH_2-O)_q-CH_2-CH_2-NH-C(=NH)-(CH_2)_3-SH$;

$-NH-(CH_2-CH_2-O)_q-CH_2-CH_2-NH-CO-(CH_2)_r-SH$;

$-NH-(CH_2-CH_2-O)_q-CH_2-CH_2-NH-CO-CH(R^{17})-CH_2-S-CH_2-CH(OH)-CH(OH)-CH_2-SH$;

$-NH-(CH_2-CH_2-O)_q-CH_2-CH_2-NH-CO-CH(NH_2)-CH_2-SH$;

$-NH-(CH_2-CH_2-O)_q-CH_2-CH_2-NH-CO-CH(NH_2)-(CH_2)_2-SH$;

$-NH-CH(CO_2H)-(CH_2)-SH$;

$-NH-CH(CO_2H)-(CH_2)_2-SH$;

and

$-NH-CH(CO_2H)-(CH_2)_2-CONH-CH(CONH-CH_2-CO_2H)-CH_2-SH$,

(wherein $R^{17}$ is a hydrogen atom or a $C_{1-6}$ alkyl group, p1 is an integer of 2 or more and 10 or less, q is an integer of 1 or more and 200 or less, and r is an integer of 1 or more and 3 or less, respectively)]
to allow chemical cross-linking to proceed within a molecule or between molecules including other molecules to form a gel.

<<Cross-linked gel of hyaluronic acid>>

The hyaluronic acid derivative according to the present embodiment can be gelated by chemical cross-linking. Namely, the cross-linked gel of the hyaluronic acid according to the present embodiment is a gel obtained by gelating the hyaluronic acid derivative according to the present embodiment by chemical cross-linking using a cross-linking agent having two or more cross-linkable groups per molecule.

The cross-linked gel of the hyaluronic acid according to the present embodiment can be obtained by gelating a liquid composition in which the hyaluronic acid derivative according to the present embodiment and a cross-linking agent are dissolved in a solvent through a chemical cross-linking reaction. In the chemical cross-linking reaction, reaction conditions such as the concentration of the hyaluronic acid derivative, the type of the cross-linking agent, the concentration of the cross-linking agent, the type of the solvent, the pH of the solvent, the concentration of the salt, the temperature, and the time can be appropriately determined. For example, the cross-linking density of the obtained gel can be increased by increasing the concentration of the cross-linking agent during chemical cross-linking, and the introduction rate of a group that can form a crosslink with the hyaluronic acid derivative or the cross-linking agent.

In the case where a cross-linking agent having groups that can form crosslinks at both ends is used, the concentration of the cross-linking agent in the step in which the hyaluronic acid derivative according to the present embodiment is gelated is preferably the concentration that allows the groups to immediately participate in the cross-linking reaction without excess or deficiency. For example, maleimide groups bond with thiol groups through a condensation reaction, and therefore when a compound having two or more thiol (SH) groups per molecule is used as a cross-linking agent to allow cross-linking to proceed by a Michael addition reaction, the ratio of maleimide groups : SH groups is preferably 3:1 to 1:3, and particularly preferably 2:1 to 1:2.

The cross-linking agent is not particularly limited as long as the cross-linking agent is a compound having two or more cross-linkable groups per molecule. The cross-linked gel of the hyaluronic acid according to the present embodiment is preferably a gel-like substance in which the hyaluronic acid derivative according to the present embodiment is chemically cross-linked using a compound having two or more thiol groups per molecule as a cross-linking agent. Examples of the compounds having two or more thiol groups per molecule include: DTT (dithiothreitol); SH-containing PEG compounds having thiol groups at both ends of a single-chain PEG; and SH-containing PEG compounds having a

branched PEG chain with 3 to 8 arms each having a thiol group at an end thereof. The molecular weight can be adjusted by adjusting the degree of polymerization of PEG of these SH-containing PEG compounds.

**In** gelation, the higher the solid concentration of the liquid composition in which the hyaluronic acid derivative and a cross-linking agent are dissolved in a solvent, the higher the osmotic pressure and the greater the tendency for the liquid composition to swell during gelation. Accordingly, when other reaction conditions are the same, the higher the concentration of the hyaluronic acid derivative, the greater the tendency for the swelling ratio of the resultant cross-linked gel to be high. Similarly, when the other reaction conditions are the same, the higher the concentration of the cross-linking agent, the greater the tendency for the swelling ratio of the resultant cross-linked gel to be high. **In** addition, when the number of cross-linkable groups per molecule of a cross-linking agent is the same, the larger the molecular weight, the greater the mass of the cross-linking agent required to add the molar amount of thiol groups required for cross-linking to the hyaluronic acid derivative, and therefore the greater the tendency for the swelling ratio to be high.

Examples of the cross-linking agent used in the production of the cross-linked gel of the hyaluronic acid according to the present embodiment include compounds containing, in the same molecule, two or more mercapto groups that react with unsaturated bonds in a nucleophilic addition reaction. Examples of the cross-linking agent include polyethylene glycol dithiol and peptides containing two or more cysteines.

The cross-linking agent used in the production of the cross-linked gel of the hyaluronic acid according to the present embodiment is preferably a 2- to 8-arm PEG compound having SH groups at ends of the arms, more preferably a 3- to 6-arm PEG compound having SH groups at ends of the arms, even more preferably a 4-arm PEG compound having SH groups at ends of the arms, even more preferably a 4-arm PEG compound having SH groups at ends of the arms with a weight-average molecular weight of 2 k to 40 k, and particularly preferably a 4-arm PEG compound having SH groups at ends of the arms with a weight-average molecular weight of 5 k to 20 k.

Although the solvent used in the step in which the hyaluronic acid derivative is gelated is preferably one that can sufficiently dissolve the hyaluronic acid derivative and the cross-linking agent, and is not particularly limited, water, dimethyl sulfoxide (DMSO), dimethylacetamide (DMAc), dimethylformamide (DMF), N-methylpyrrolidone (NMP), or a mixture of the solvents selected therefrom is preferably used.

Alternatively, an organic solvent that is miscible with these solvents may be mixed and used. Although there is no particular limitation, examples of the miscible organic solvent include methanol, ethanol, propanol, isopropanol, butanol, polyhydric alcohols, acetone, and acetonitrile. Examples of the polyhydric alcohols include the same ones as those mentioned above, and among them, ethylene glycol is preferable.

In addition, a basic compound is preferably added to improve the stability of protein or peptide and to increase the reaction rate during the cross-linking reaction. Although the basic compound used is not particularly limited, examples thereof include: carbonates such as sodium carbonate and sodium bicarbonate; hydroxides such as sodium hydroxide; aqueous ammonia; and amines such as pyridine, triethylamine, ethylenediamine, ethanolamine, diethanolamine, and triethanolamine. Amines are preferably used, and triethanolamine is more preferably used.

Since the hyaluronic acid derivative forms nanoparticles in an aqueous solution as targeting carriers, nano-sized microparticle gels can be formed by allowing cross-linking to proceed under dilute conditions, and can be used as sustained release carriers in the blood. The dilute conditions are 10 mg/mL or less, preferably 5 mg/mL or less, and more preferably 1 mg/mL or less. On the other hand, bulk gels in which microparticles are cross-linked with each other can be formed by allowing cross-linking to proceed under high concentration conditions. The bulk gels are useful as subcutaneous sustained-release carriers. The high concentration conditions are 5 mg/mL or more, preferably 20 mg/mL or more, and more preferably 30 mg/mL.

The step in which the hyaluronic acid derivative is gelated may be carried out in bulk or in a discontinuous phase such as an emulsion or spray droplets. For example, in the case where the step is carried out in a W/O emulsion, an aqueous phase in which the hyaluronic acid derivative and a cross-linking agent are dissolved may be emulsified in a solvent which is not miscible in water to allow the gelation reaction to proceed. Although the solvent which is not miscible in water is not particularly limited, examples thereof include hexane, chloroform, dichloromethane, ethyl acetate, medium-chain triglyceride (MCT), liquid paraffin, and soybean oil. A surfactant which stabilizes emulsion may be added. **In** addition, the step may be carried out in a solvent that allows desolvation, such as supercritical carbon dioxide or PEG. **In** this case, once an aqueous phase or organic solvent phase in which the hyaluronic acid derivative, a cross-linking agent and the like are dissolved is emulsified and dispersed in the above-mentioned solvent, the polymer is concentrated due to desolvation (solvent diffusion), thereby obtaining a gel with a higher cross-linking density.

During or after the step in which the hyaluronic acid derivative is gelated, an operation in which the cross-linking reaction is terminated, and an operation in which remaining cross-linkable groups are inactivated or washed away may be carried out. Cross-linkable groups that do not participate in the reaction, groups bonded with only one end of the cross-linking agent molecules, remaining cross-linking agent molecules, and the like are preferably removed from the standpoint of safety, storage stability, side reactions with a drug to be encapsulated, or the like. Although there is no particular limitation, when unreacted cross-linking agent molecules remain, they may be removed by washing with an excessive amount of water, for example.

The step in which the hyaluronic acid derivative is gelated may be followed by a grinding step. Although examples of the grinding method include grinding using a pestle and a mortar, and grinding using a mill, grinding using a mill is preferable. Although examples of a mill grinder include: rotating disk type grinders such as a centrifugal mill (manufactured by NIHONSEIKI KAISHA LTD.) and an impact mill (manufactured by DALTON CORPORATION); screen mill grinders such as an atomizer (manufactured by TOKYO ATOMIZER MFG. CO., LTD.), a sample mill (manufactured by TOKYO ATOMIZER MFG. CO., LTD.), a bantam mill (manufactured by TOKYO ATOMIZER MFG. CO., LTD.) and an SK mill (manufactured by Tokken. Inc.); jet mills such as an ultra-micro labo jet mill (A-O jet mill, manufactured by SEISHIN ENTERPRISE CO., LTD.); and a Linrex mill (manufactured by LIQUID GAS Co., Ltd.) which allows grinding at ultra-low temperatures, a SK mill and a Linrex mill are preferable.

The cross-linked gel of the hyaluronic acid according to the present embodiment may be a gel-like substance obtained by chemically cross-linking the hyaluronic acid derivative according to the present embodiment with a cross-linking agent, or a dried substance obtained by drying the gel-like substance. Examples of the drying method include freeze drying, ventilation drying, drying in a constant temperature bath, vacuum drying, and hot air circulation type drying. The wind speed, drying time, temperature, pressure and the like are appropriately selected within a range in which the gel of the hyaluronic acid derivative is not decomposed or deteriorated.

The dried substance of the cross-linked gel of the hyaluronic acid according to the present embodiment and the cross-linked gel of the hyaluronic acid according to the present embodiment are porous structures in both the dried state and the swollen state. Accordingly, relatively large components such as cells can be supported, for example, and the dried substance can be suitably used as a base material of cells and tissues used in tissue engineering and the like. Cells for transplantation can be attached and cultured in the dried substance, and then transplanted into the target tissue, for example. The dried substance and the cross-linked gel also serve as scaffolds on which cells growth in situ regeneration therapy. For example, cells that have migrated from the surrounding tissue can be attached using the dried substance as a scaffold by embedding the dried substance in tissue within the body.

The dried substance of the cross-linked gel of the hyaluronic acid according to the present embodiment and the cross-linked gel of the hyaluronic acid according to the present embodiment can control the adhesiveness of cells by changing the introduction rate of the cholesteryl derivative.

<<Pharmaceutical composition>>

A pharmaceutical composition according to the present embodiment contains the above-mentioned hyaluronic acid derivative. **In** the pharmaceutical composition according to the present embodiment, the hyaluronic acid derivative mainly serves as a carrier of an active ingredient. When the pharmaceutical composition is administered to the living body, an active ingredient such as a drug is gradually released from the carrier containing the hyaluronic acid derivative, and favorable sustained-releaseability can be expected.

<Active ingredient>

The active ingredient contained in the pharmaceutical composition according to the present embodiment is not particularly limited as long as it is a substance that is used as a drug for humans or animals (substances administered for the diagnosis, treatment, or prevention of a disease) or a substance used as an active ingredient thereof. The active ingredient may be a water-soluble substance or a poorly soluble substance. Examples of the active ingredient include proteins, peptides, polysaccharides, oligosaccharides, nucleic acids, oligonucleotides, low molecular weight compounds, and cells. **In** addition, the active ingredient may be a complex of protein and nucleic acid, such as a Cas9/gRNA complex.

Examples of the pharmaceutical composition according to the present embodiment include nanoparticles formed from the hyaluronic acid according to the present embodiment and encapsulating an active ingredient, the dried substance of the cross-linked gel of the hyaluronic acid according to the present embodiment, the cross-linked gel of the hyaluronic acid to which cells for transplantation have been engrafted, the cross-linked gel of the hyaluronic acid in which a poorly soluble drug is encapsulated, and dried substances thereof.

<Form>

The pharmaceutical composition according to the present embodiment may be a dispersible fine particle solution, a precipitation suspension, or a freeze-dried product. **In** the case of the freeze-dried product, the pharmaceutical composition may be a precipitation-type sustained-release preparation to which an isotonic solution such as physiological saline is added by a doctor to prepare an administration solution before administration. **In** this case, the pharmaceutical composition is considered to be suitable for containing an active ingredient that is unstable in a solution state.

When the pharmaceutical composition according to the present embodiment is a dispersible fine particle solution or a precipitation suspension, the concentration of the hyaluronic acid derivative in the pharmaceutical composition is

preferably 1 mg/mL or more and 200 mg/mL or less, more preferably 4 mg/mL or more and 100 mg or less, even more preferably 4 mg/mL or more and 50 mg/mL or less, and particularly preferably 4 mg/mL or more and 12 mg/mL or less.

The pharmaceutical composition according to the present embodiment is not limited to the forms which have been already mentioned, and may be in the form of nanoparticles, microparticles, solutions, emulsions, suspensions, gels, micelles, implants, powders, or films. Powders may be produced by grinding solids obtained by freeze-drying or spray-drying, or may be produced from dried precipitates.

The pharmaceutical composition according to the present embodiment may be administered orally, parenterally, intranasally, intravaginally, intraocularly, subcutaneously, intravenously, intramuscularly, intradermally, intraperitoneally, intracerebrally, or intraorally. **In** addition, the pharmaceutical composition according to the present embodiment is not limited to an injection, and may be a patch preparation, a microneedle preparation, a topical ointment, an eye drop, a spray, an inhalant, or the like.

[Example]

Although the present invention will be described in detail below with reference to examples, it is not intended to limit the scope of the present invention to the examples.

The methods for measuring and evaluating physical properties of hyaluronic acid derivatives produced in Examples and Comparative Examples are described below. Unless otherwise specified, all experiments were carried out in triplicate.

<$^1$H-NMR measurement>

$^1$H-NMR measurement was carried out using 0.02 N DCl/d6-DMSO as a solvent and a JEOL JNM-A400 spectrometer (manufactured by JEOL Ltd.) equipped with a 400 MHz-NMR instrument (JNM-ECS400, manufactured by JEOL Ltd.), unless otherwise stated.

<Measurement by size exclusion chromatography coupled with multi-angle laser scattering>

HA derivatives were analyzed by SEC-MALS using a Wyatt Dawn NEON multi-angle light scattering detector and an Optilab refractive index detector equipped with an isocratic HPLC system (manufactured by Waters Corporation). First, the HA derivatives were diluted to 1.0 mg/mL with a SEC-MALS buffer (10 mM phosphate buffer, pH 7.4). A separation step was carried out in the SEC-MALS buffer using a G4000SWXL column (manufactured by Tosoh) at a flow rate of 0.5 mL/min. Data analysis was carried out using the refractive index concentration coefficient dn/dc value of hyaluronic acid (0.153, literature value) and Astra software (manufactured by Wyatt Technology Corporation).

<Measurement of average particle diameter by dynamic light scattering (DLS) method>

The HA derivatives were characterized by DLS analysis using an ELSZ-2000 (manufactured by Otsuka Electronics Co., Ltd.) in solution of the same solvent as SEC (10 mM phosphate buffer, pH 7.4). The measured autocorrelation function was analyzed by the cumulant method. The hydrodynamic diameter of the HA derivatives was analyzed using the Stokes-Einstein equation.

<Inverted vial test (test of gelation by cross-linking HA derivative)>

Cross-linked gels of HA derivative were prepared by allowing cross-linking to proceed through a Michael addition reaction between maleimide groups and thiol groups. A PEG compound having SH groups at ends of four arms thereof under the tradename of 4arm-PEGSH (molecular weight = $1\times10^4$ g/mol, manufactured by NOF CORPORATION) was used as a cross-linking agent to cause gelation, unless otherwise specified.

The gelationability of the HA derivative was analyzed based on the state when a vial was inverted. Specifically, a HA derivative and PEGSH were completely dissolved separately in each solvent (10 mM phosphate buffer, pH 7.4), and then both of the solutions were poured into a single vial while cooling to 5°C, and then incubated at 37°C for 30 minutes. The vial was inverted over time from the start of incubation to check whether a sample flowed and dropped. In the case in which the sample did not flow within 5 minutes or 30 minutes from the start of incubation, the sample was classified into gel. In the case in which the sample flowed even after 30 minutes from the start of incubation, the sample was classified into sol. All samples were classified into either gel or sol.

<Preparation of cross-linked gel of HA derivative>

Two types of precursor solution (a phosphate buffer solution of HA derivative and a phosphate buffer solution of PEGSH) were poured into a disk-shaped silicone rubber mold (having a diameter of 6 mm and a depth of 1 mm) placed on a polytetrafluoroethylene membrane while cooling to 5°C. The mold was then covered with a silicone cover sheet and incubated at 37°C for 30 minutes. After incubation, the sample was transferred to a glass vial. The molar ratio between maleimide groups and thiol groups was 1.1:1.

<Swelling of hydrogel>

Cross-linked gels of a HA derivative (φ = 6 mm, thickness = 1.0 mm) were placed at the bottom of pre-weighed double glass vials (n=4). The initial weight of each vial containing the cross-linked gel of the HA derivative was measured as the initial mass. Then, 1 mL of PBS was added thereto and incubated at 37°C. At 1, 12, and 24 hours, the buffer was carefully removed from each vial and the vial was weighed to measure the swollen mass. Fresh PBS was then added to replace the removed solution. The mass of the original hydrogel ($W_0$) and the mass of the swollen hydrogel ($W_S$) were calculated by subtracting the mass of an empty vial from the total mass. The mass swelling ratio (Q) of the cross-linked gel of the HA derivative was calculated by dividing the swollen mass by the initial mass.

[Mathematical Formula 1]

$$Q = W_s / W_0 \times 100\ (\%)$$

<Experiment of uptake or release of protein into or from cross-linked gel of HA derivative>

A cross-linked gel of a HA derivative (φ = 6 mm, thickness = 10 mM) was prepared in the same manner as mentioned above and immersed in PBS. After reaching equilibrium swelling, the cross-linked gel of the HA derivative was immersed in a PBS solution of FITC-insulin (100 μg/mL) at 37°C. Then, a sample (200 μL of solution) was collected at a specific point on the gel, and its fluorescence intensity was measured using a DeNovix DS-11 FX+ fluorescence spectrophotometer ("Fluoroprotein" module). The loading efficiency of FITC-insulin was estimated from the decrease in fluorescence intensity.

An in vitro release test of FITC-insulin from a HA derivative in the presence of serum was also evaluated. A cross-linked gel of the HA derivative (φ = 6 mm, thickness = 1 mm) complexed with FITC-insulin was immersed in PBS (pH 7.4) containing 1 mL of 10% fetal bovine serum (FBS) at 37°C. Samples (200 μL of the gel supernatant) were then collected at specific times, and the fluorescence intensity and the loading efficiency of FITC-insulin were measured as described above.

[Example 1]

A HA derivative (HA-C6-Chol-Male, hereinafter, may be referred to as "HAMICH") in which steryl groups and maleimide groups were introduced into at least a part of carboxyl groups in glucuronic acid moieties of the hyaluronic acid was prepared.

[Chemical Formula 5]

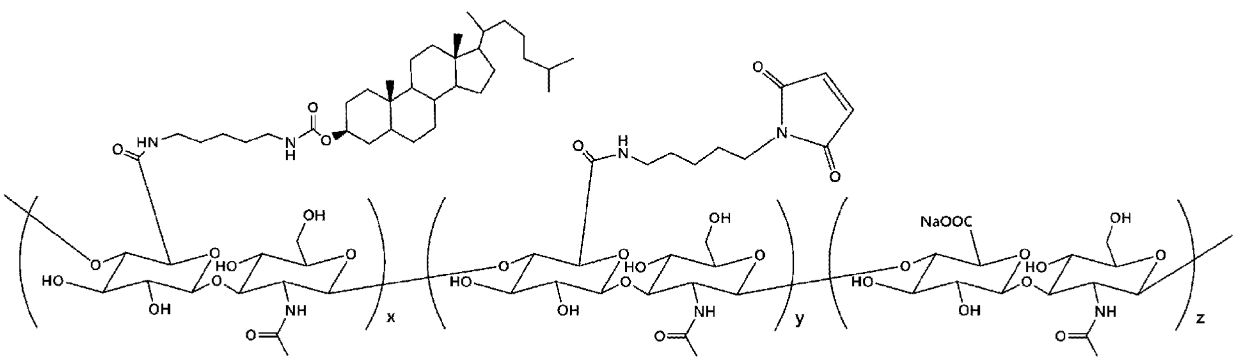

Cholesteryl 6-aminohexylcarbamate hydrochloride (Chol-C6 hydrochloride) and a tetrabutylammonium salt of hyaluronic acid (HA-TBA) were synthesized as described in Example 1 and Example 2 of Patent Document 1.

Specifically, the HA-TBA was obtained by converting HA from sodium hyaluronate (HA-Na) (average molecular weight = 120 kDa, purchased from Bloomage Biotechnology Japan) as a starting material using a cation exchange resin (Dowex (registered trademark) 50WX-8-400, manufactured by Sigma-Aldrich), and reacting the resultant with N-(5-aminopentyl)maleimide hydrochloride using 4-(4,6-dimethoxy-1,3,5-triazin-2-yl)-4-methylmorpholinium chloride (DMT-MM) (manufactured by KOKUSAN CHEMICAL Co., Ltd.) as a condensing agent.

The HA-TBA was completely dissolved in DMSO (1% w/v), and then N-(5-aminopentyl)maleimide dissolved in DMSO was added thereto, followed by stirring the mixture at room temperature for 5 min. Subsequently, the DMT-MM was added to the mixture and stirred at room temperature overnight. The feed molar ratio was 100:20:24 (glucuronic acid of HA: DMT-MM: N-(5-aminopentyl)maleimide hydrochloride, x=20).

Cholesteryl-6-aminohexylcarbamate was then reacted by the same procedure, at the feed molar ratio of 100:x:1.2x (glucuronic acid of HA: DMT-MM: cholesteryl-6-aminohexylcarbamate, x=1.2, 5, 10, 16, 22, 31, or 44).

The obtained reaction solution was dialyzed (Spectrapore 4, molecular weight cut-off (MWCO): 12 kDa to 14 kDa, manufactured by Spectrum Laboratories) against a DMSO solution, an aqueous solution of 0.15 M NaCl, and ultrapure water in this order, and the obtained dialysate was freeze-dried to obtain each target product (HA-C6-Chol-1%-Male 15% to HA-C6-Chol-40%-Male 15%) shown in Table 1 as a white solid.

As a comparative control, a hyaluronic acid derivative modified with maleimide groups but unmodified with Chol was produced in the same manner as mentioned above, except that Chol hydrochloride was not reacted. The target product (HA-C6-Chol-0%-Male) shown in Table 1 was obtained as a white solid.

[Table 1]

| HA-C6-Chol-Male (HAMICH) | Addition molar ratio between N-(5-aminopentyl)maleimide hydrochloride and DMT-MM (HA-TBA units/ N-(5-aminopentyl)maleimide hydrochloride/ DMT-MM) 15% | Addition molar ratio between Chol-C6 hydrochloride and DMT-MM (HA-TBA units /Chol-C6 hydrochloride/ DMT-MM) 15% |
|---|---|---|
| HA-C6-Chol-0%-Male | | - |
| HA-C6-Chol-1%-Male15% | | 100/1.2/1.44 |
| HA-C6-Chol-5%-Male15% | | 100/5/6 |
| HA-C6-Chol-10%-Male15% | | 100/10/12 |
| HA-C6-Chol-15%-Male15% | 100/20/24 | 100/16/19.2 |
| HA-C6-Chol-20%-Male15% | | 100/22/26.4 |
| HA-C6-Chol-30%-Male15% | | 100/31/37.2 |
| HA-C6-Chol-40%-Male15% | | 100/44/52.8 |

The structure of each synthesized product was confirmed by $^{1}$H-NMR measurement. HAMICH dissolved in 0.02N DC1/DMSO was used to carry out NMR analysis. As a result of $^{1}$H-NMR measurement, the degree of maleimidation (introduction rate of maleimide groups) of each HA derivative was 15%. Each NMR chart of the synthesized product is shown in FIG. 1A (Chol-0%) to FIG. 1H (Chol-40%).

The z-average particle diameter (nm) of each HAMICH was measured. The measurement results are shown in Table 2. In addition, the degree of association of each HAMICH was calculated from the absolute molecular weight measured by GPC-MALS analysis and the theoretical average molecular weight per unit. The results are shown in Table 2.

[Table 2]

| HA-C6-Chol-Male (HAMICH) | z-Average particle diameter [nm] | Number of association of HAMICH |
|---|---|---|
| HA-C6-Chol-0%-Male | 178.4 | - |
| HA-C6-Chol-1 %-Male15% | 145.5 | 7 |
| HA-C6-Chol-5%-Male15% | 105.5 | 29 |
| HA-C6-Chol-10%-Male15% | 72.8 | 68 |
| HA-C6-Chol-15%-Male15% | 36.9 | 13 |

(continued)

| HA-C6-Chol-Male (HAMICH) | z-Average particle diameter [nm] | Number of association of HAMICH |
|---|---|---|
| HA-C6-Chol-20%-Male 15% | 29.0 | 7 |
| HA-C6-Chol-30%-Male15% | 34.3 | 22 |
| HA-C6-Chol-40%-Male15% | 161.2 | 1773 |

In the HAMICH, the particle size decreased depending on the introduction rate of cholesteryl groups up to 20%, but when the introduction rate of cholesteryl groups increased to 40%, the particle size increased, which indicates that the introduction rate of cholesteryl groups affects the particle size of nanoparticles of HA derivative. Furthermore, in the HAMICH unmodified with cholesteryl (0%), there was almost no association because the hydrophobic interactions of steryl groups did not work.

[Example 2]

Each HAMICH prepared in Example 1 was chemically cross-linked using 4arm-PEGSH as a cross-linking agent to prepare a cross-linked gel of the HAMICH.

First, each HAMICH prepared in Example 1 was dissolved in 10 mM phosphate buffer (pH 7.4) to the concentrations shown in Table 3 to prepare a HAMICH solution. In a separate container, 4arm-PEGSH (Mw: 10,000) was dissolved in 10 mM phosphate buffer (pH 7.4) to a concentration of 20 mg/mL to prepare a PEGSH solution. After cooling both of the solutions to 5°C, they were mixed in a silicon mold having a thickness of 1 mm and a diameter of 6 mm such that the molar ratio between maleimide groups with which the hyaluronic acid was modified and SH groups derived from the 4arm-PEGSH was 1.1:1.0 to produce a cross-linked gel of the HAMICH at 37°C. All cases were carried out under conditions that makes the final concentration of the hyaluronic acid derivative be 7 mg/mL.

[Table 3]

| HA-C6-Chol-Male (HAMICH) | Concentration of HAMICH solution [ mg/mL] |
|---|---|
| HA-C6-Chol-0%-Male | 30 |
| HA-C6-Chol-1%-Male15% | 25 |
| HA-C6-Chol-5%-Male 15% | 10 |
| HA-C6-Chol-10%-Male15% | 15 |
| HA-C6-Chol-15%-Male15% | 10 |
| HA-C6-Chol-20%-Male15% | 25 |
| HA-C6-Chol-30%-Male15% | 25 |
| HA-C6-Chol-40%-Male15% | 30 |

All eight types of HAMICH into which cholesteryl groups and maleimide groups were introduced were sufficiently gelated to form cross-linked gels within 1 to 30 minutes from the start of gelation (mixing of the HAMICH solution and the PEGSH solution), regardless of the introduction rate of cholesteryl groups. As shown in Comparative Example 2 below, in HAMA (HA-C6-Chol-3%-MA, and HA-C6-Chol-19%-MA) into which methacryl groups were introduced, gelation was difficult with the hyaluronic acid derivatives into which steryl groups and methacryl groups were introduced. It was revealed from these results that introducing a combination of steryl groups and maleimide groups into hyaluronic acid made rapid gelation possible, such a rapd gelation being difficult with the hyaluronic acid derivatives into which steryl groups and methacryl groups were introduced.

In addition, the change in volume in PBS after gelation was measured to examine the swelling ratio. The measurement results are shown in FIG. 2 (A). Furthermore, HA-C6-Chol-0%-Male and HAMICH (HA-C6-Chol-20%-Male) in which the introduction rate of cholesteryl group was 20% were subjected to gelation reactions under the same conditions except that the final concentration of the hyaluronic acid derivative was 16.7 mg/mL, and the change in volume from the start of gelation was measured to examine the swelling ratio thereof. The measurement results are shown in FIG. 2 (B). As shown in FIG. 2, swelling of gel was observed over time in HA-C6-Chol-0%-Male unmodified with cholesteryl groups. In contrast, HAMICH modified with both maleimide groups and steryl groups exhibited characteristic properties in which the swelling ratio was smaller than that of HAMICH modified only with maleimide groups, and an increase in volume after

gelation in vivo was suppressed.

[Comparative Example 1]

A HA derivative (HA-C6-Chol-MA, which may be referred to as "HAMA") in which steryl groups and methacryl groups were introduced into at least a part of carboxyl groups in glucuronic acid moieties of a hyaluronic acid was prepared.

[Chemical Formula 6]

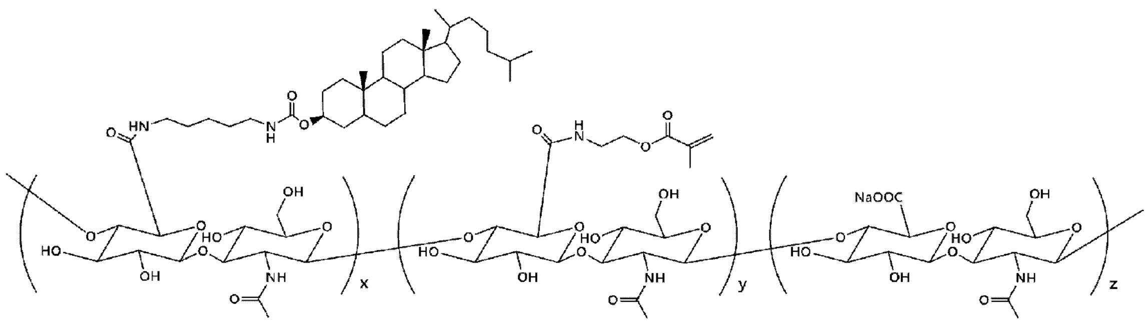

Cholesteryl 6-aminohexylcarbamate hydrochloride (Chol-C6 hydrochloride) and tetrabutylammonium salt of hyaluronic acid (HA-TBA) were synthesized as described in Example 1 and Example 2 of Patent Document 1.

Specifically, HA-TBA was obtained by converting HA from sodium hyaluronate (HA-Na) (average molecular weight = 120 kDa, purchased from Bloomage Biotechnology Japan) as a starting material using a cation exchange resin (Dowex (registered trademark) 50WX-8-400, manufactured by Sigma-Aldrich), and reacting the resultant with 2-aminoethyl methacrylate hydrochloride (manufactured by FUJIFILM Wako Pure Chemical Corporation, product number 510-51961) using 4-(4,6-dimethoxy-1,3,5-triazin-2-yl)-4-methylmorpholinium chloride (DMT-MM) (manufactured by KOKUSAN CHEMICAL Co., Ltd.) as a condensing agent.

HA-TBA was completely dissolved in DMSO (1% w/v), and then 2-aminoethyl methacrylate hydrochloride (manufactured by FUJIFILM Wako Pure Chemical Corporation, product number 510-51961) dissolved in DMSO was added thereto, followed by stirring the mixture at room temperature for 5 minutes. DMT-MM was then added to the mixture and stirred overnight at room temperature. The feed molar ratio was 100:20:24 (glucuronic acids of HA: DMT-MM: 2-aminoethyl methacrylate hydrochloride, x=20).

Cholesteryl-6-aminohexylcarbamate was then reacted by the same procedure, at the feed molar ratio of 100:x:1.2x (glucuronic acids of HA: DMT-MM: cholesteryl-6-aminohexylcarbamate, x=2.0, 19).

The HA product was purified by dialysis (MWCO: 12 to 14 kDa) against DMSO, then against 0.150 M NaCl, and finally against distilled water. The purified HA solution was filtered through a 0.22 μm membrane filter and freeze-dried to dryness.

Then, 2-aminoethyl methacrylate hydrochloride was added to each solution at the ratio relative to HA-TBA units shown in Table 4. Next, 4-(4,6-dimethoxy-1,3,5-triazin-2-yl)-4-methylmorpholinium chloride (DMT-MM) (manufactured by KOKUSAN CHEMICAL Co., Ltd.) was added to each solution at the ratio relative to HA-TBA units shown in Table 4, and stirred overnight. Then, Chol-C6 hydrochloride was added to each solution at the ratio relative to HA-TBA units shown in Table 4.

Next, DMT-MM was added at the ratio relative to HA-TBA units shown in Table 4, and the mixture was further stirred overnight at room temperature. Finally, the reaction solution was dialyzed (Spectrapore 4, molecular weight cutoff (MWCO): 12 kDa to 14 kDa, manufactured by Spectrum Laboratories) against a DMSO solution, an aqueous solution of 0.15 M NaCl, and ultrapure water, in this order, and the resultant dialysate was freeze-dried to obtain the target product (HA-C6-Chol-MA) shown in Table 4 as a white solid.

[Table 4]

| HA-C6-Chol-MA (HAMA) | Addition molar ratio between 2-aminoethyl methacrylate hydrochloride and DMT-MM (HA-TBA units/ 2-aminoethyl methacrylate hydrochloride/ DMT-MM) 15% | Addition molar ratio between Chol-C6 hydrochloride and DMT-MM (HA-TBA units/ Chol-C6 hydrochloride/ DMT-MM) 15% |
|---|---|---|
| HA-C6-Chol-3%-MA20% | 100/20/24 | 100/2/2.4 |

(continued)

| HA-C6-Chol-MA (HAMA) | Addition molar ratio between 2-aminoethyl methacrylate hydrochloride and DMT-MM (HA-TBA units/ 2-aminoethyl methacrylate hydrochloride/ DMT-MM) 15% | Addition molar ratio between Chol-C6 hydrochloride and DMT-MM (HA-TBA units/ Chol-C6 hydrochloride/ DMT-MM) 15% |
|---|---|---|
| HA-C6-Chol-19%-MA19% | 100/20/24 | 100/19/24.7 |

[Comparative Example 2]

Each HAMA prepared in Comparative Example 1 was chemically cross-linked using 4arm-PEGSH as a cross-linking agent to obtain a cross-linked gel of the HAMA.

First, each HAMA prepared in Comparative Example 1 was dissolved in 10 mM phosphate buffer (pH 7.4) to the concentrations shown in Table 5 to prepare a HAMA solution. In a separate container, 4arm-PEGSH (manufactured by NOF CORPORATION, Mw: 10,000) was dissolved in 10 mM phosphate buffer (pH 7.4) to a concentration of 50 mg/mL to prepare a PEGSH solution. The both solutions were cooled to 5°C, and then mixed in a silicon mold having a thickness of 1 mm and a diameter of 6 mm such that the molar ratio between methacryl groups with which the hyaluronic acid was modified and SH groups derived from the 4arm-PEGSH was 1.1:1.0, thereby producing a cross-linked gel of the HAMA at 37°C. All cases were carried out under conditions that make the final concentration of the hyaluronic acid derivative be 7 mg/mL.

[Table 5]

| HA-C6-Chol-MA (HAMA) | Concentration of HAMA solution [mg/mL] |
|---|---|
| HA-C6-Chol-3%-MA20% | 25 |
| HA-C6-Chol-19%-MA19% | 50 |

Each HAMA in which methacrylic groups were introduced (HA-C6-Chol-3%-MA and HA-C6-Chol-19%-MA) was still insufficiently gelated 30 minutes after the start of gelation, and gelation was not completed even 24 hours after the start of gelation.

[Comparative Example 3]

Furthermore, in a manner similar to that of Comparative Example 2, HAMA (HA-C6-Chol-19%-MA) in which methacrylic groups were introduced was tried to be cross-linked using 4arm-PEGSH (manufactured by NOF CORPORATION, Mw: 10,000) dissolved at a concentration of 100 mg/mL under the same conditions except that the final concentration of the hyaluronic acid derivative was 25 mg/mL. As a result, the HAMA was not cross-linked even under the high concentration conditions.

[Example 3]

Fluorescently labeled insulin (FITC-insulin) was uptaken in the cross-linked gel of the HAMICH produced in Example 2 to examine the uptake ratio.

Each cross-linked gel of HAMICH (having a diameter of 6 mm and a thickness of 1 mm) produced under the conditions that made the final concentration of the hyaluronic acid derivative was 7 mg/mL in Example 2 was immersed in 1 mL of a FITC-insulin solution (a solution in which 250 μg/mL of FITC-insulin was dissolved in PBS) and incubated at 25°C, and the fluorescence intensity of the supernatant thereof was quantified over time to calculate the uptake ratio (%) of the FITC-insulin in the gel. The uptake ratios (%) 1 hour, 8 hours, and 24 hours after the start of incubation are shown in Table 6 and FIG. 3. In addition, the appearance of each gel after the uptake is also shown in FIG. 4.

[Table 6]

| HA-C6-Chol-Male (HAMICH) | Uptake ratio [%] 1 hour after incubation | Uptake ratio [%] 8 hours after incubation | Uptake ratio [%] 24 hours after incubation |
|---|---|---|---|
| HA-C6-Chol-0%-Male15% | 1.9 | 2.2 | 0.3 |

(continued)

| HA-C6-Chol-Male (HAMICH) | Uptake ratio [%] 1 hour after incubation | Uptake ratio [%] 8 hours after incubation | Uptake ratio [%] 24 hours after incubation |
|---|---|---|---|
| HA-C6-Chol-1%-Male15% | 3.4 | 6.2 | 7.4 |
| HA-C6-Chol-5%-Male15% | 4.7 | 14.7 | 18.5 |
| HA-C6-Chol-10%-Male15% | 4.2 | 10.2 | 18.7 |
| HA-C6-Chol-15%-Male15% | 2.7 | 8.7 | 11.9 |

As shown in Table 6, the cross-linked gels of the HAMICH into which cholesteryl groups were introduced exhibited higher uptake ratio of FITC-insulin than the cross-linked gel of the HAMICH into which no cholesteryl groups were introduced. In addition, the uptake ratio of FITC-insulin was also affected by the introduction rate of cholesteryl groups, and the best uptake ratio after incubation for 8 and 24 hours was achieved when the introduction rate of cholesteryl groups was 5 to 10%. It was also clear from visual inspection of FIG. 4 that the cross-linked gel into which no cholesteryl groups were introduced was weakly colored by FITC. It was revealed from these results that the hyaluronic acid derivative according to the present embodiment exhibits excellent uptake efficiency of an active ingredient such as protein, and a larger amount of an active ingredient can be uptaken by optimizing the introduction rate of steryl groups.

As shown in the results of Examples 2 and 3, the use of the hyaluronic acid derivative according to the present embodiment makes it possible to achieve rapid gelation and furthermore to obtain a novel cross-linked gel that can simultaneously suppress the change in volume in vivo and uptake a large amount of protein.

In view of the above, the cross-linked gel obtained from the hyaluronic acid derivative according to the present embodiment makes it possible to encapsulate a large amount of cytokines required for regeneration or cell differentiation, and is expected to be applied to tissue engineering usage in which cell adhesion and differentiation are controlled by the release of these cytokines.

[Example 4]

Cross-linked gels of hyaluronic acid derivatives modified with both maleimide groups and steryl groups were produced to examine whether the cross-linked gels became porous even in the swelling equilibrium state after freeze-drying and freeze-thawing.

First, a hyaluronic acid derivative modified with a fluorescent Cy5 (Cy5-HAMICH) in which the introduction rate of cholesteryl groups was 19% and the introduction rate of maleimide groups was 11% was produced in the same manner as in Example 1. The modification with the Cy5 was carried out by adding, relative to the HA-TBA units, 0.02 equivalents of Cy5-amine (manufactured by Funakoshi Co., Ltd., product number: BP-22559) and 0.03 equivalents of DMT-MM as a condensation agent. In addition, a hyaluronic acid derivative modified with a fluorescent Cy5 (Cy5-HAMICH) in which no cholesteryl groups were introduced (the introduction rate thereof was 0%) and the introduction rate of maleimide groups was 7% was also produced in the same manner as above. Next, each Cy5-HAMICH was gelated by adding a cross-linking agent thereto such that the ratio of maleimide groups: SH groups was 1.1:1.0 (molar ratio) in the same manner as in Example 3, thereby producing a cross-linked gel (having a diameter of 8 mm and a thickness of 1 mm). After the production, the gel was left to stand in PBS for 1 hour and then subjected to a freeze-thaw treatment.

The freeze-thaw treatment was carried out as follows. First, the cross-linked gel of the HA derivative modified with the Cy5 was left still at room temperature (25°C) to 5°C for 30 minutes, then left still at -20°C for 1 hour, and then left still at -78°C for 24 hours to carry out the freeze-dry treatment. Then, the resultant was thawed by incubating it in a water bath at 25°C.

Fluorescence microscopy images of each cross-linked gel after the freeze-dry treatment and thawing treatment were obtained using a two-photon laser microscope (Zeiss). Fluorescence microscopy images of each cross-linked gel are shown in FIG. 5. It was confirmed that the cross-linked gel of the Cy5-HAMICH in which the introduction rate of cholesteryl groups was 19% (upper row of FIG. 5) was a porous structure in which numerous pores having a diameters of several tens of μm formed on the surface thereof like a mesh structure. In contrast, no such pores were confirmed in the cross-linked gel of the Cy5-HAMICH in which the introduction rate of cholesteryl groups was 0% (lower row of Figure, 5). It was considered from these results that the cross-linked gel of the hyaluronic acid derivative according to the present embodiment is useful as a scaffold in which cells infiltrate and promote regeneration.

[Example 5]

In the same manner as in Example 2, the HAMICH prepared in Example 1 was chemically cross-linked using

4arm-PEGSH as a cross-linking agent to prepare a cross-linked gel of the HAMICH.

First, each HAMICH produced in Example 1 was dissolved in 10 mM phosphate buffer (pH 7.4) to a concentration of 10 mg/mL to prepare a HAMICH solution. Only HA-C6-Chol-20%-Male 15% was dissolved to a concentration of 25 mg/mL. In a separate container, 4arm-PEGSH (Mw: 10,000) was dissolved in 10 mM phosphate buffer (pH 7.4) to a concentration of 20 mg/mL to prepare a PEGSH solution. After cooling both solutions to 5°C, they were poured into a single vial and mixed, and an inverted vial test was carried out to verify the gel or sol. At this time, 10 mM phosphate buffer (pH 7.4) was appropriately added to the 4arm-PEGSH solution before mixing the hyaluronic acid derivative and the 4arm-PEGSH solution such that the final concentrations of the hyaluronic acid derivative and the 4arm-PEGSH were as shown in Table 7.

[Table 7]

| Test Plot | HA-C6-Chol-Male (HAMICH) | Final concentration of HAMICH [ mg/mL] | Final concentration of 4arm-PEGSH [ mg/mL] |
|---|---|---|---|
| 5-1 | HA-C6-Chol-1 %-Male15% | 2.00 | 0.53 |
| 5-2 | | 2.85 | 0.57 |
| 5-3 | | 2.85 | 2.27 |
| 5-4 | | 3.50 | 1.40 |
| 5-5 | | 3.50 | 2.79 |
| 5-6 | | 5.00 | 1.99 |
| 5-7 | HA-C6-Chol-5%-Malel5% | 1.90 | 0.31 |
| 5-8 | | 1.90 | 0.71 |
| 5-9 | | 1.90 | 1.42 |
| 5-10 | | 2.85 | 0.27 |
| 5-11 | | 2.85 | 0.53 |
| 5-12 | | 2.85 | 1.06 |
| 5-13 | | 2.85 | 2.12 |
| 5-14 | | 2.85 | 4.67 |
| 5-15 | | 3.80 | 0.31 |
| 5-16 | | 3.80 | 0.62 |
| 5-17 | | 3.80 | 1.42 |
| 5-18 | | 3.80 | 2.83 |
| 5-19 | HA-C6-Chol-10%-Male15% | 2.56 | 0.93 |
| 5-20 | | 2.56 | 1.86 |
| 5-21 | | 3.40 | 0.54 |
| 5-22 | | 3.41 | 1.24 |
| 5-23 | | 3.41 | 2.48 |
| 5-24 | HA-C6-Chol-15%-Male15% | 4.90 | 1.61 |
| 5-25 | | 4.90 | 3.23 |
| 5-26 | | 6.53 | 2.15 |
| 5-27 | | 6.53 | 4.31 |
| 5-28 | HA-C6-Chol-20%-Male15% | 10.0 | 4.99 |
| 5-29 | | 10.0 | 6.53 |
| 5-30 | | 12.5 | 6.23 |

Each HAMICH, into which cholesteryl groups and maleimide groups were introduced, was gelated sufficiently and formed a cross-linked gel within 1 to 30 minutes from the start of gelation (mixing of the HAMICH solution and the

PEGSH solution) in all test plots 5-1 to 5-30, regardless of the introduction rate of cholesteryl groups. It was revealed from these results that hyaluronic acid derivatives in which steryl groups and maleimide groups were introduced into hyaluronic acid in combination could be gelated at various concentrations. In addition, it was suggested that the uptake ratio of drug in the resultant cross-linked gel of the HAMICH could be changed depending on the gel concentration.

[Example 6]

In the same manner as in Example 5, each HAMICH prepared in Example 1 was chemically cross-linked using 4arm-PEGSH as a cross-linking agent to prepare a cross-linked gel of the HAMICH.

First, HAMICH (HA-C6-Chol-15%-Male15%) produced in Example 1 such that the introduction rate of cholesteryl groups was 15% was dissolved in 10 mM phosphate buffer (pH 7.4) to a concentration of 10 mg/mL to prepare a HAMICH solution. In a separate container, 4arm-PEGSH (Mw: 10,000) was dissolved in 10 mM phosphate buffer (pH 7.4) to a concentration of 70 mg/mL to prepare a PEGSH solution. After cooling both solutions to 5°C, they were poured into a single vial and mixed, and the gel or sol was verified by an inverted vial test. At this time, 10 mM phosphate buffer (pH 7.4) was added appropriately to the 4arm-PEGSH solution before mixing the hyaluronic acid derivative and the 4arm-PEGSH solution such that the final concentrations of the hyaluronic acid derivative and the 4arm-PEGSH were as shown in Table 8.

[Table 8]

| Test Plot | HA-C6-Chol-Male (HAMICH) | Final concentration of HAMICH [ mg/mL] | Final concentration of 4arm-PEGSH [ mg/mL] |
|---|---|---|---|
| 6-1 | HA-C6-Chol-15%-Male15% | 9.00 | 3.73 |
| 6-2 | | 9.00 | 6.66 |

Each HAMICH into which 15% of cholesteryl groups and 15% of maleimide groups were introduced was gelated sufficiently and formed a cross-linked gel 30 minutes after the start of gelation (mixing of the HAMICH solution and the PEGSH solution) in both test plots 6-1 and 6-2. It was revealed from these results demonstrated that hyaluronic acid derivatives in which steryl groups and maleimide groups are introduced into hyaluronic acids in combination could be gelated at various concentrations. In addition, it was suggested that the uptake ratio of drug in the resultant cross-linked gel of the HAMICH could be changed depending on the gel concentration.

[Example 7]

The hyaluronic acid derivative modified with both maleimide groups and steryl groups was analyzed by gel permeation chromatography with multi-angle light scattering (GPC-MALS) to calculate the absolute molecular weight of the aggregate in 10 mM phosphate buffer (pH 7.4).

MALS detectors measure scattering signals from polymers or particles in a sample at different scattering angles $\theta$. The basic light scattering equation (Anderson et al, Analytical Chemistry, 2003, vol. 75, p. 4279-4291) is shown below. In the equation, $R_\theta$ is the excess Rayleigh ratio, K is an optical constant that especially depends on the specific refractive index increment (dn/dc), c is the solute concentration, M is the molecular weight, Rg is the radius of gyration, and $\lambda$ is the wavelength of the incident light. Calculation of molecular weight and radius of gyration from light scattering data requires extrapolation to zero angle (Wyatt, Analytica Chimica Acta, 1993, vol. 272(1), p. 1-40). This is done by plotting $(Kc/R_\theta)^{1/2}$ as a function of $\sin^2(\theta/2)$ in a so-called Debye plot. In general, the molecular weight can be calculated from the intercept of the ordinate, and the radius of gyration can be calculated from the initial slope of the curve.

[Mathematical Formula 2]

$$\sqrt{\frac{Kc}{R_\theta}} = \sqrt{\frac{1}{M} + \frac{16\pi^2}{3\lambda^2}\frac{1}{M}Rg^2\sin^2\left(\frac{\theta}{2}\right)},$$

In this study, signals from the RI detector and the MALS detector were collected, and calculation and analysis were carried out using a software of ASTRA8 (manufactured by Wyatt Technology Corporation). The calculated molecular weight, for example, the absolute weight-average molecular weight Mw (absolute value), was obtained using a refractive

index concentration coefficient dn/dc of 0.153, which is the literature value of hyaluronic acid. The measurement conditions and calculation results are shown below.

(Measurement conditions)

Apparatus: SEC-Multi Detector System
Column: G4000SWXL (manufactured by Tosoh)
MALS: DWAN NEON (manufactured by Wyatt Technology Corporation)
Laser wavelength: 661 nm
RI: Optilab (manufactured by NEON)
Eluent: 10mM PB, pH 7.4
Flow rate: 0.5 mL/min
Injection volume: 50 μL
Concentration: 1.0mg/mL
Temperature: 25°C
Analysis software: ASTRA8 (manufactured by Wyatt Technology Corporation))

[Table 9]

| Test plot | HA-C6-Chol-Male (HAMICH) | Mw (Molecular weight) by SEC-MALS [kDa] |
|---|---|---|
| 7-1 | HA-C6-Chol-0%-Male15% | 298.7 |
| 7-2 | HA-C6-Chol-1%-Male15% | 853.3 |
| 7-3 | HA-C6-Chol-5%-Male 15% | 3864.8 |
| 7-4 | HA-C6-Chol-10%-Male15% | 9619.1 |
| 7-5 | HA-C6-Chol-15%-Male15% | 1947.3 |
| 7-6 | HA-C6-Chol-20%-Malel5% | 1126.8 |
| 7-7 | HA-C6-Chol-30%-Male15% | 3682.4 |
| 7-8 | HA-C6-Chol-40%-Male15% | 310944 |

It is suggested from these results that cholesterol is the driving force behind the formation of an aggregate, and that an aggregate of a hyaluronic acid derivative modified with cholesterol and maleimide groups can be formed even in the absence of a cross-linking agent. It is expected that effects of gelation and cross-linking reactions on a drug can be suppressed by preliminary encapsulating the drug in nanoparticles composed of an aggregate of a hyaluronic acid derivative and then carrying out a cross-linking reaction to cause gelation. This method makes it possible to obtain a cross-linked gel in which the drug is encapsulated without impairing the stability of the drug, even in the case in which the drug is highly reactive with a cross-linking agent. It was thought that such an encapsulation of a drug in an aggregate of a hyaluronic acid derivative could be applied to a drug with a small molecular weight, such as insulin, as well as a cytokine with a large molecular weight, such as IL-4.

[Example 8]

An insulin release test was carried out in vitro on each cross-linked gel of the HAMICH obtained in Example 3 in which a fluorescently labeled insulin (FITC-insulin) was uptaken.

Each of the five types of the resultant cross-linked gel of HAMICH obtained in Example 3 in which the FITC-insulin was uptaken was immersed and incubated in 1 mL of PBS (pH 7.4) containing 10% FBS at 37°C, and the fluorescence intensity in the supernatant was quantified over time to calculate the release ratio (%) of FITC-insulin from each gel. FIG. 6 shows the results of the release ratio (%) from the start of immersion to 21 days thereafter of the cross-linked gels of the HAMICHs modified with cholesteryl groups (HA-C6-Chol-1%-Male 15%, HA-C6-Chol-5%-Male 15%, HA-C6-Chol-10%-Male 15%, and HA-C6-Chol-20%-Male 15%).

In the case of the cross-linked gel unmodified with cholesterol (HA-C6-Chol-0%-Male 15%), the entire amount of uptaken FITC-insulin was released into the supernatant (release rate 100%) one day after the start of immersion. In

contrast, as shown in FIG. 6, in the case of the cross-linked gels of the HAMICHs modified with cholesteryl groups, the release ratio of the FITC-insulin did not reach 100% even 21 days after the start of immersion, and the sustained release of FITC-insulin continued for a long period of time. It was revelaed from these results that the cross-linked gel obtained from the hyaluronic acid derivative according to the present embodiment not only exhibits excellent uptake efficiency of an active ingredient such as protein, but also has excellent sustained-release properties.

As shown by the above-mentioned examples, the cross-linked gel obtained from the hyaluronic acid derivative according to the present embodiment is gelated quickly and the change in volume thereof in vivo is suppressed. In addition, the cross-linked gel can encapsulate a large amount of an active ingredient such as protein and exhibits excellent long-term sustained release properties of the encapsulated substance. In view of these, the hyaluronic acid derivative according to the present embodiment and the cross-linked gel obtained therefrom are expected to be applied to tissue engineering usage. The cross-linked gel obtained from the hyaluronic acid derivative according to the present embodiment can encapsulate a large amount of cytokines necessary for regeneration or cell differentiation, and the subsequent release thereof makes it possible to control cell adhesion and differentiation.

[Example 9]

The target product (HA-C6-Chol-1%-Male 8.1%) of Test plot 9-1 shown in Table 10 was obtained as a white solid in the same manner as in Example 1, except that sodium hyaluronate (HA-Na) having a weight-average molecular weight of 35 kDa (purchased from Bloomage Biotechnology Japan) was used as a starting material sodium hyaluronate (HA-Na). The structure of the resultant synthesized product was confirmed by $^1$H-NMR measurement. As a result, the introduction rate of Chol was 1.3% and the introduction rate of maleimide groups was 8.1% in the HA derivative of Test plot 9-1.

In addition, sodium hyaluronate (HA-Na) having a weight average molecular weight of 35 kDa was used as a raw material, and cholesteryl groups and methacryl groups are introduced thereinto in the same manner as in Comparative Example 1 with the composition shown in Table 10, followed by modifying the resultant with maleimide groups in the same manner as in Example 1 to obtain the target product (HA-C6-Chol-17%-Male 4.6%) of Test plot 9-2 shown in Table 10 as a white solid. The structure of the resultant synthesized product was confirmed by $^1$H-NMR measurement. As a result, the introduction rate of Chol was 17.4%, the introduction rate of maleimide groups was 4.6%, and the introduction rate of methacryl groups was 9.7% in the HA derivative of Test plot 9-2.

[Table 10]

| Test plot | HA-C6-Chol-Male (HAMICH) | Addition molar ratio between N-(5-aminopentyl) maleimide hydrochloride and DMT-MM (HA-TBA units/ N-(5-aminopentyl) maleimide hydrochloride/ DMT-MM) | Addition molar ratio between Chol-C6 hydrochloride and DMT-MM (HA-TBA units/ Chol-C6 hydrochloride/ DMT-MM) | Addition molar ratio between 2-aminoethyl methacrylate hydrochloride and DMT-MM (HA-TBA units/ 2-aminoethyl methacrylate hydrochloride/ DMT-MM) |
|---|---|---|---|---|
| 9-1 | HA-C6-Chol-1 %-Male 8.1% | 100/20/24 | 100/2/2.4 | - |
| 9-2 | HA-C6-Chol-17%-Male 4.6% | 100/10/12 | 100/19/24.7 | 100/10/12 |

[Example 10]

The HAMICH prepared in Example 9 was chemically cross-linked using 4arm-PEGSH as a cross-linking agent to prepare a cross-linked gel of the HAMICH.

First, the HAMICH of Test plot 9-1 was dissolved in 10 mM phosphate buffer (pH 7.4) to a concentration of 14 mg/mL to prepare a HAMICH solution. In a separate container, 4arm-PEGSH (Mw: 10,000) was dissolved in 10 mM phosphate buffer (pH 7.4) to a concentration of 7 mg/mL to prepare a PEGSH solution. After the both solutions were cooled to 5°C, they were mixed in a silicon mold having a thickness of 1 mm and a diameter of 6 mm such that the molar ratio between maleimide groups with which the hyaluronic acid was modified and SH groups derived from the 4arm-PEGSH was 1.1:1.0, thereby producing a cross-linked gel of the HAMICH at 37°C. This was carried out under conditions that made the final concentration of the hyaluronic acid derivative be 7 mg/mL.

Similarly, the HAMICH of Test plot 9-2 was dissolved in 10 mM phosphate buffer (pH 7.4) to a concentration of 30 mg/mL to prepare a HAMICH solution. In a separate container, 4arm-PEGSH (Mw: 10,000) was dissolved in 10 mM phosphate buffer (pH 7.4) to a concentration of 13 mg/mL to prepare a PEGSH solution. After both solutions were cooled to 5°C, they were mixed in a silicon mold having a thickness of 1 mm and a diameter of 6 mm such that the molar ratio between maleimide groups with which the hyaluronic acid was modified and SH groups derived from 4arm-PEGSH was 1.1:1.0 to produce a cross-linked gel of the HAMICH at 37°C. This was carried out under conditions that made the final concentration of the hyaluronic acid derivative be 20 mg/mL.

The HAMICH, into which cholesteryl groups and maleimide groups were introduced, was gelated sufficiently and formed a cross-linked gel within 1 to 30 minutes from the start of gelation (mixing of the HAMICH solution and the PEGSH solution), regardless of the introduction rate of maleimide groups. Although it was predicted that a decrease in the introduction rate of maleimide groups decreased the number of cross-linked points, thereby making gelation more difficult, it was revealed from these results that introducing steryl groups and maleimide groups in combination into a hyaluronic acid enabled rapid gelation, which was difficult to achieve with hyaluronic acid derivatives into which steryl groups and methacryl groups were introduced, regardless of the introduction rate of maleimide groups.

Furthermore, the change in volume in PBS from the start of gelation was measured to examine the swelling ratio. The measurement results of the HAMICH in Test plot 9-1 are shown in FIG. 7(A), and the measurement results of the HAMICH in Test plot 9-2 are shown in FIG. 7(B). As shown in FIG. 7, the HAMICH modified with both maleimide groups and steryl groups exhibited characteristics properties in which the swelling ratio thereof was small regardless of the introduction rate of maleimide groups, and the change in volume thereof in vivo after the gelation was suppressed.

[Comparative Example 4]

The gelation of hyaluronic acid derivatives unmodified with maleimides was investigated.

First, a hyaluronic acid derivative unmodified with maleimide was synthesized. Specifically, sodium hyaluronate (HA-Na) having a weight-average molecular weight of 35 kDa or 100 kDa was used as a raw material, and cholesteryl groups were introduced thereinto in the same manner as in Example 1 with the composition shown in Table 11, thereby obtaining the target product of Comparative test plot 4-1 (HA-C6-Chol-19%) and the target product of Comparative test plot 4-2 (HA-C6-Chol-40%) as white solids, respectively. Cholesteryl 6-aminohexyl carbamate hydrochloride (Chol-C6 hydrochloride) and tetrabutylammonium hyaluronate (HA-TBA) were synthesized as described in Examples 1 and 2 of Patent Document 1. As a result, the target products (HA-C6-Chol-19% and HA-C6-Chol-40%) shown in Table 11 were obtained as white solids.

[Table 11]

| C. test plot | Average molecular weight [kDa] of HA raw material | HA-C6-Chol | Addition molar ratio between Chol-C6 hydrochloride and DMT-MM (HA-TBA units/ Chol-C6 hydrochloride/ DMT-MM) | Addition molar ratio between N-(5-aminopentyl) maleimide hydrochloride and DMT-MM (HA-TBA units/ N-(5-aminopentyl) maleimide hydrochloride/ DMT-MM) |
|---|---|---|---|---|
| 9-1 | 35 | HA-C6-Chol-19% | 100/19/22.8 | - |
| 9-2 | 100 | HA-C6-Chol-40% | 100/44/52.8 | - |
| (C. test plot: Comparative test plot) | | | | |

Next, each of the HA derivative of Comparative test plot 4-1 (HA-C6-Chol-19%) and the HA derivative of Comparative test plot 4-2 was chemically cross-linked using 4arm-PEGSH as a cross-linking agent to prepare a cross-linked gel of the HA derivative (unmodified with maleimide).

First, each HA derivative (unmodified with maleimide) produced in Comparative test plots 4-1 and 4-2 was dissolved in 10 mM phosphate buffer (pH 7.4) to a concentration of 14 mg/mL to prepare a HA derivative solution. **In** a separate container, 4arm-PEGSH (Mw: 10,000) was dissolved in 10 mM phosphate buffer (pH 7.4) to a concentration of 7 mg/mL to prepare a PEGSH solution. After both solutions were cooled to 5°C, they were mixed in a silicon mold having a thickness of 1 mm and a diameter of 6 mm such that the molar ratio between the molecular weight of the hyaluronic acid derivative and SH groups derived from the 4arm-PEGSH was 1.1:1.0, to prepare a cross-linked gel at 37°C. This was carried out under conditions that made the final concentration of the hyaluronic acid derivative be 7 mg/mL. As a result, gelation did not occur even after 24 hours. Similarly, it was confirmed that no gelation occurred in the inverted vial test of the both Comparative test plots.

INDUSTRIAL APPLICABILITY

The hyaluronic acid derivative according to the present embodiment makes it possible to provide a hyaluronic acid derivative that decreases the change in volume after gelation and realizes sustained-release of an active ingredient.

## Claims

1. A hyaluronic acid derivative having steryl groups introduced into at least a part of carboxyl groups in glucuronic acid moieties of a hyaluronic acid and maleimide groups introduced into at least a part of carboxyl groups in glucuronic acid moieties of the hyaluronic acid or hydroxyl groups in N-acetylglucosamine moieties thereof.

2. The hyaluronic acid derivative according to claim 1, wherein the maleimide groups are introduced into the at least a part of carboxyl groups in the glucuronic acid moieties of the hyaluronic acid.

3. The hyaluronic acid derivative according to claim 1, wherein a total of an introduction rate of the steryl groups and an introduction rate of the maleimide groups relative to the hyaluronic acid derivative is less than 45%.

4. The hyaluronic acid derivative according to claim 1, wherein an introduction rate of the steryl groups relative to the hyaluronic acid derivative is 0.5% or more and 30% or less.

5. The hyaluronic acid derivative according to claim 1, wherein an introduction rate of the maleimide groups relative to the hyaluronic acid derivative is 1.0% or more and 25% or less.

6. The hyaluronic acid derivative according to claim 1, wherein the hyaluronic acid derivative has at least one repeating unit of general formula (I) shown below:

[Chemical Formula 1]

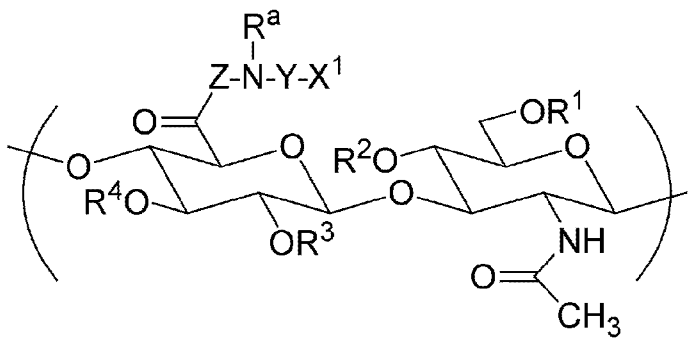

(I)

[in the general formula (I), $R^1$, $R^2$, $R^3$, and $R^4$ are each independently a group selected from the group consisting of a hydrogen atom, $C_{1-6}$ alkyls, a formyl and $C_{1-6}$ alkylcarbonyls;
Z is a direct bond or a peptide linker consisting of 2 to 30 arbitrary amino acid residues;
$X^1$ is a group selected from the group consisting of $-NR^b-R$, $-NR^b-COO-R$, $- NR^b-CO-R$, $-NR^b-CO-NR^c-R$, -COO-R, -O-COO-R, -S-R, $-CO-Y^a-S-R$, $-O-CO-Y^b-S-R$, $-NR^b-CO-Y^b-S-R$, and -S-S-R;
$R^a$, $R^b$ and $R^c$ are each independently a group selected from the group consisting of a hydrogen atom, $C_{1-20}$ alkyls, amino $C_{2-20}$ alkyls and hydroxy $C_{2-20}$ alkyls, and a group selected from the group consisting of -O- and $-NR^f-$ may be introduced in an alkyl moiety of $R^a$, $R^b$ or $R^c$ mentioned above;
$R^f$ is selected from the group consisting of a hydrogen atom, $C_{1-12}$ alkyls, amino $C_{2-12}$ alkyls and hydroxy $C_{2-12}$ alkyls, and a group selected from the group consisting of - O- and -NH- may be introduced in an alkyl moiety of $R^f$;
R is a steryl group;
Y is a $C_{2-30}$ alkylene or $-(CH_2CH_2O)_m-CH_2CH_2-$, and a group selected from the group consisting of -O-, $-NR^g-$ and -S-S- may be introduced in an alkylene moiety of Y;
$R^g$ is a group selected from the group consisting of a hydrogen atom, $C_{1-20}$ alkyls, amino $C_{2-20}$ alkyls and hydroxy $C_{2-20}$ alkyls, and a group selected from the group consisting of -O- and -NH- may be introduced in an alkyl moiety of $R^g$;

$Y^a$ is a $C_{1-5}$ alkylene;
$Y^b$ is a $C_{2-8}$ alkylene or a $C_{2-8}$ alkenylene; and
m is an integer of 1 or more and 100 or less], and
at least one repeating unit of general formula (II) shown below:

[Chemical Formula 2]

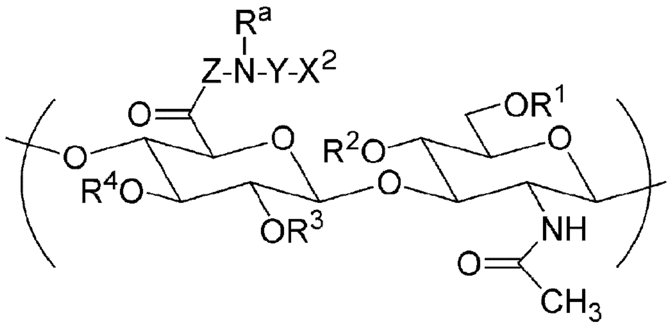

(II)

[In general formula (II), $R^1$, $R^2$, $R^3$, and $R^4$ are each independently a group selected from the group consisting of a hydrogen atom, $C_{1-6}$ alkyls, a formyl and $C_{1-6}$ alkylcarbonyls;
Z is a direct bond or a peptide linker composed of 2 to 30 arbitrary amino acid residues;
$X^2$ is a maleimide group;
$R^a$ is a group selected from the group consisting of a hydrogen atom, $C_{1-20}$ alkyls, amino $C_{2-20}$ alkyls and hydroxy $C_{2-20}$ alkyls, and a group selected from the group consisting of -O- and $-NR^f-$ may be introduced in an alkyl moiety of $R^a$;
Y is a $C_{2-30}$ alkylene or $-(CH_2CH_2O)_m-CH_2CH_2-$, and a group selected from the group consisting of -O-, $-NR^g-$ and -S-S- may be introduced in an alkylene moiety of Y;
$R^g$ is a group selected from the group consisting of a hydrogen atom, $C_{1-20}$ alkyls, amino $C_{2-20}$ alkyls and hydroxy $C_{2-20}$ alkyls, and a group selected from the group consisting of -O- and -NH- may be introduced in an alkyl moiety of $R^g$; and
m is an integer of 1 or more and 100 or less].

**7.** The hyaluronic acid derivative according to claim 1, wherein the steryl groups are cholesteryl groups.

**8.** The hyaluronic acid derivative according to claim 1, wherein an average particle diameter of the hyaluronic acid derivative when dissolved in a phosphate buffer solution (10 mM) at a concentration of 1 mg/mL is 250 nm or less, the average particle diameter being calculated by a dynamic light scattering method.

**9.** A method for producing a cross-linked gel of a hyaluronic acid derivative, comprising reacting a hyaluronic acid derivative of any one of claims 1 to 8 with a cross-linking agent having two or more cross-linkable groups to be gelated.

**10.** The method for producing a cross-linked gel of a hyaluronic acid derivative according to claim 9, wherein the cross-linkable groups are thiol groups.

**11.** A cross-linked gel of a hyaluronic acid derivative, wherein the cross-linked gel is a gel-like substance in which the hyaluronic acid derivative of any one of claims 1 to 8 is chemically cross-linked with a cross-linking agent having two or more cross-linkable groups,.

**12.** The cross-linked gel of a hyaluronic acid derivative according to claim 11, wherein the cross-linkable groups are thiol groups.

**13.** The cross-linked gel of a hyaluronic acid derivative according to claim 11, wherein a swelling ratio is 115% or less.

**14.** A dried substance of a cross-linked gel of a hyaluronic acid derivative, wherein the dried substance is a dried substance of a gel-like substance in which a hyaluronic acid derivative of any one of claims 1 to 8 is chemically cross-linked with a cross-linking agent having two or more cross-linkable groups.

**15.** The dried substance of a cross-linked gel of a hyaluronic acid derivative according to claim 14, wherein the dried substance is a porous structure.

**16.** A pharmaceutical composition comprising a hyaluronic acid derivative of any one of claims 1 to 8.

**17.** A pharmaceutical composition comprising a cross-linked gel of a hyaluronic acid derivative, wherein the cross-linked gel is a gel-like substance in which a hyaluronic acid derivative of any one of claims 1 to 8 is chemically cross-linked with a cross-linking agent having two or more cross-linkable groups.

**18.** The pharmaceutical composition according to claim 17, further comprising an active ingredient.

FIG. 1A

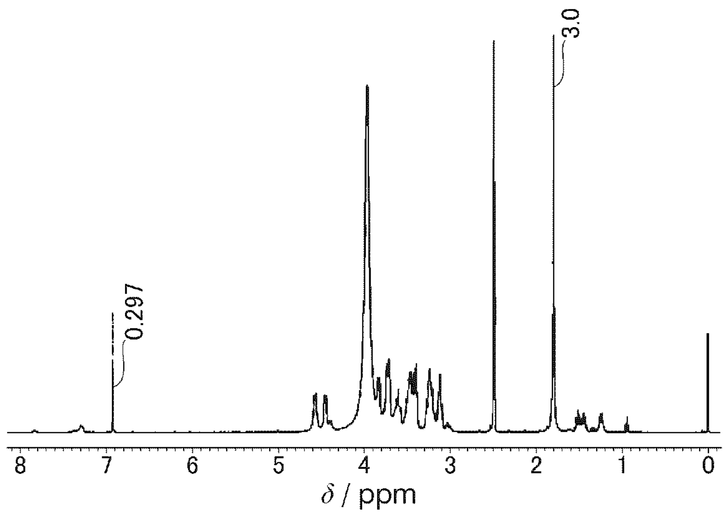


FIG. 1B

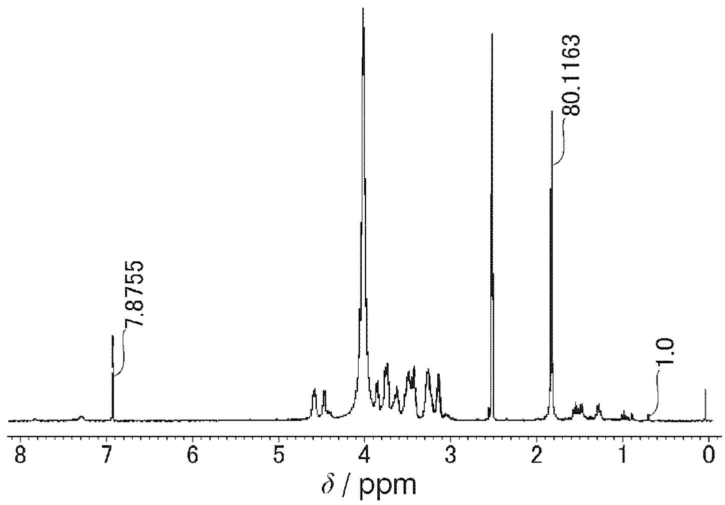


FIG. 1C

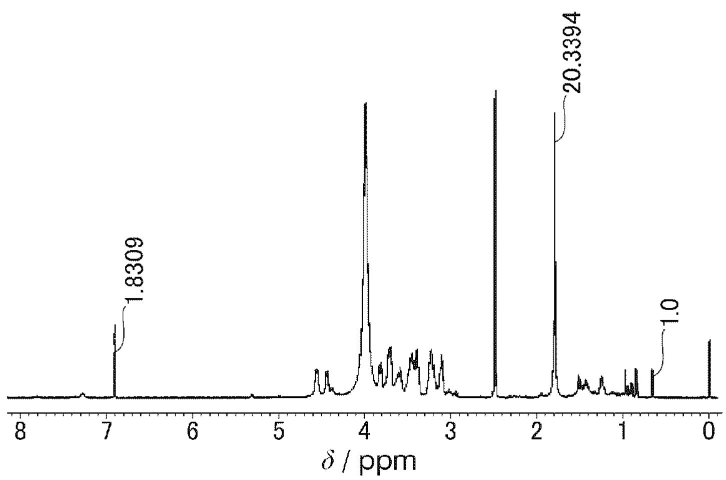

FIG. 1D

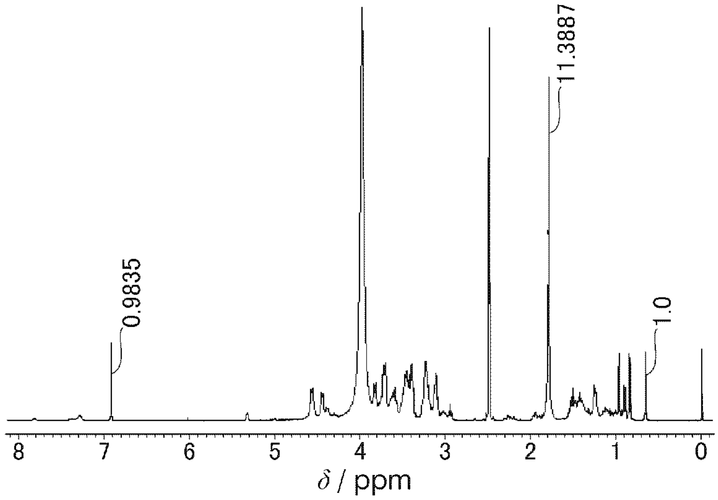


FIG. 1E

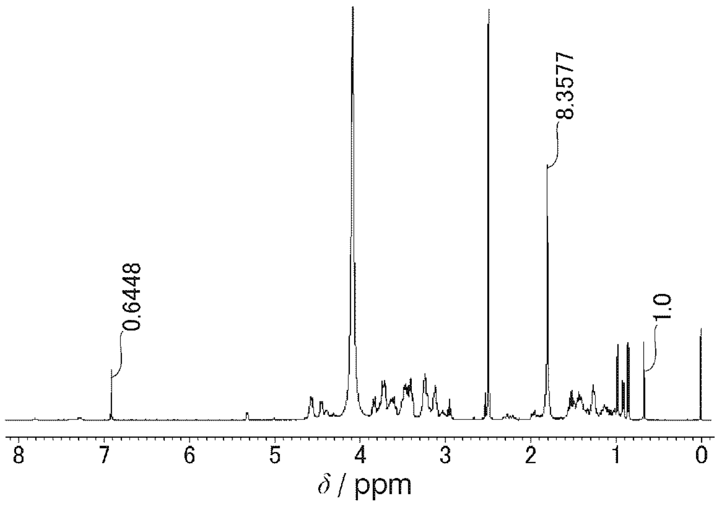


FIG. 1F

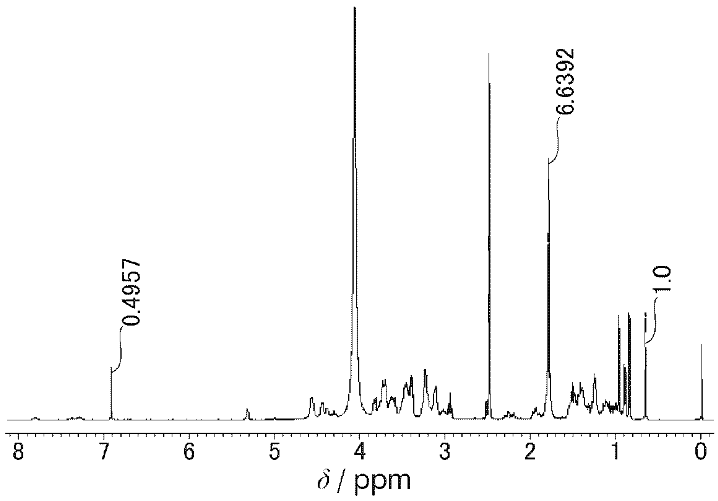

*FIG. 1G*

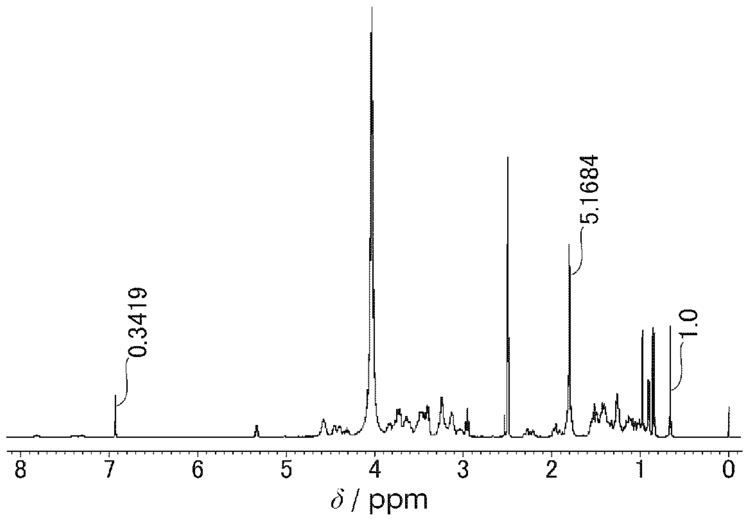
0.3419
5.1684
1.0
8
7
6
5
4
3
2
1
0
δ / ppm

*FIG. 1H*

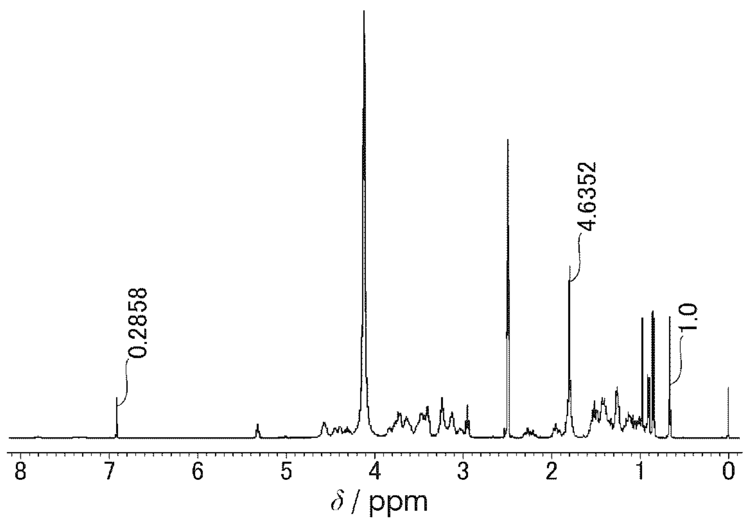
0.2858
4.6352
1.0
8
7
6
5
4
3
2
1
0
δ / ppm

FIG. 2

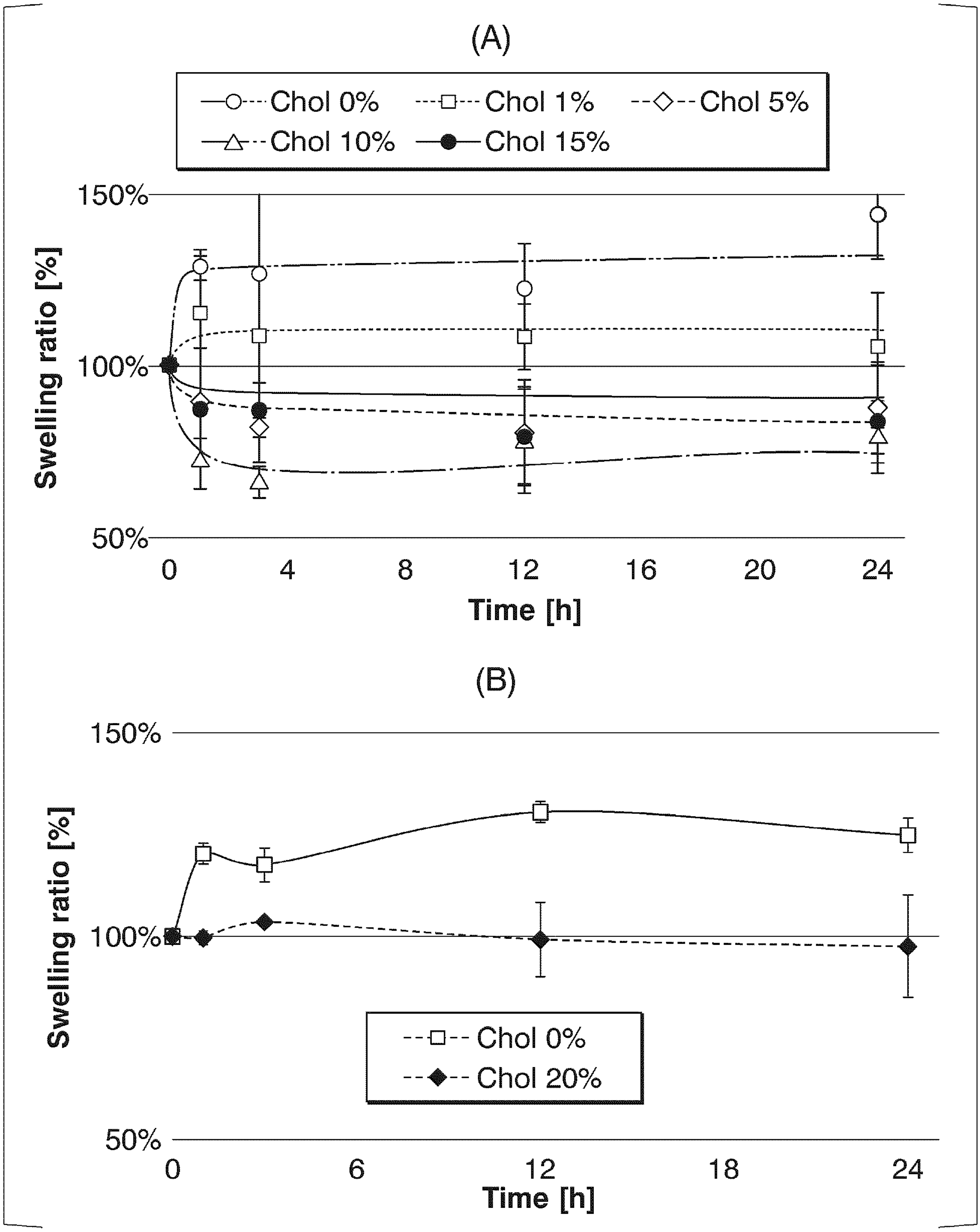
(A)
Chol 0%
Chol 1%
Chol 5%
Chol 10%
Chol 15%
150%
100%
50%
Swelling ratio [%]
0
4
8
12
16
20
24
Time [h]
(B)
150%
100%
50%
Swelling ratio [%]
Chol 0%
Chol 20%
0
6
12
18
24
Time [h]

FIG. 3

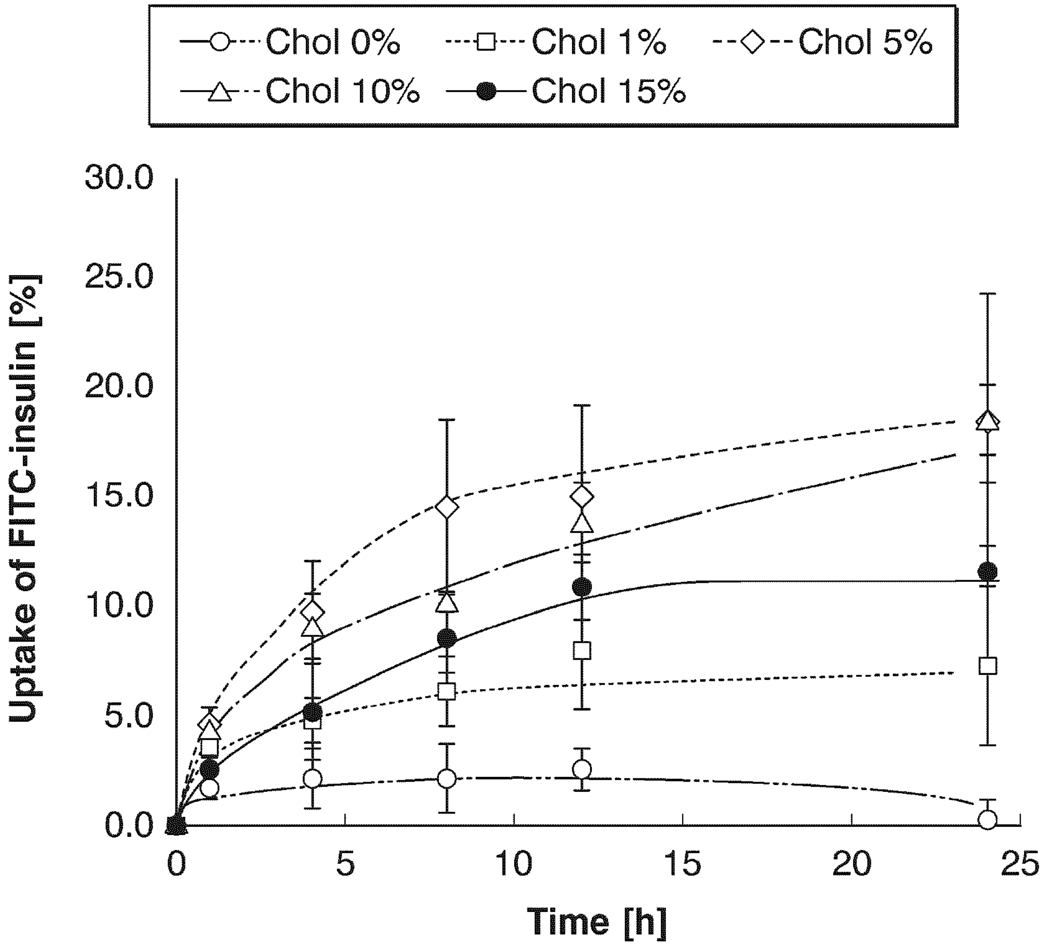
Chol 0%
Chol 1%
Chol 5%
Chol 10%
Chol 15%
Uptake of FITC-insulin [%]
30.0
25.0
20.0
15.0
10.0
5.0
0.0
0
5
10
15
20
25
Time [h]

FIG. 4

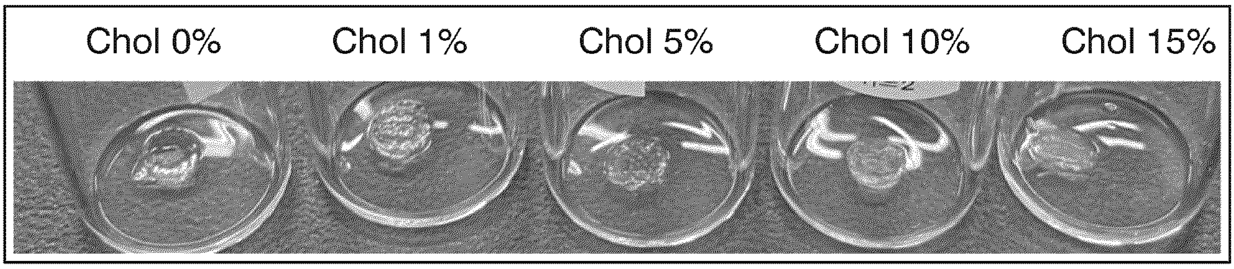
Chol 0%
Chol 1%
Chol 5%
Chol 10%
Chol 15%

*FIG. 5*

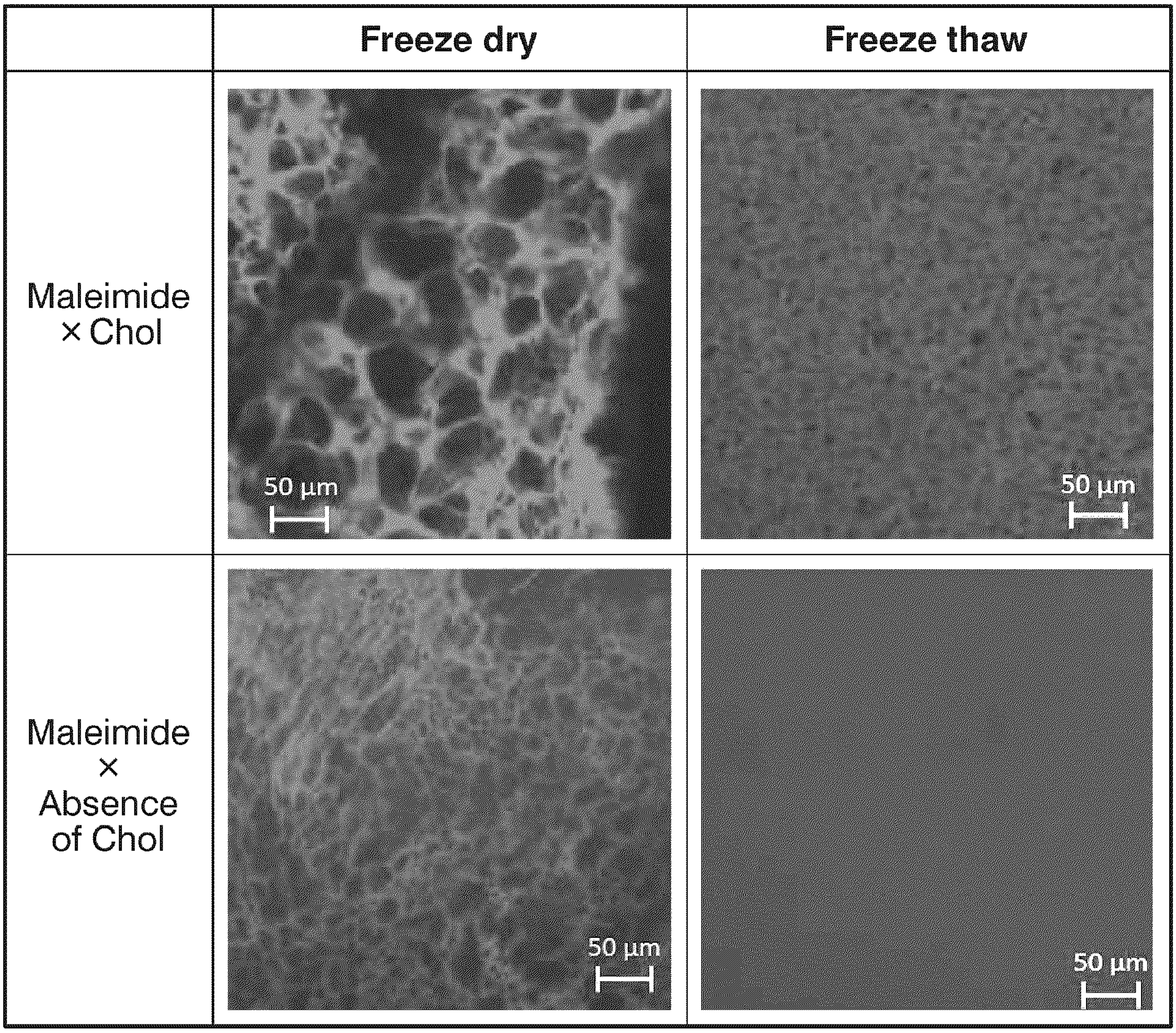
Freeze dry
Freeze thaw
Maleimide × Chol
Maleimide × Absence of Chol
50 µm
50 µm
50 µm
50 µm

*FIG. 6*

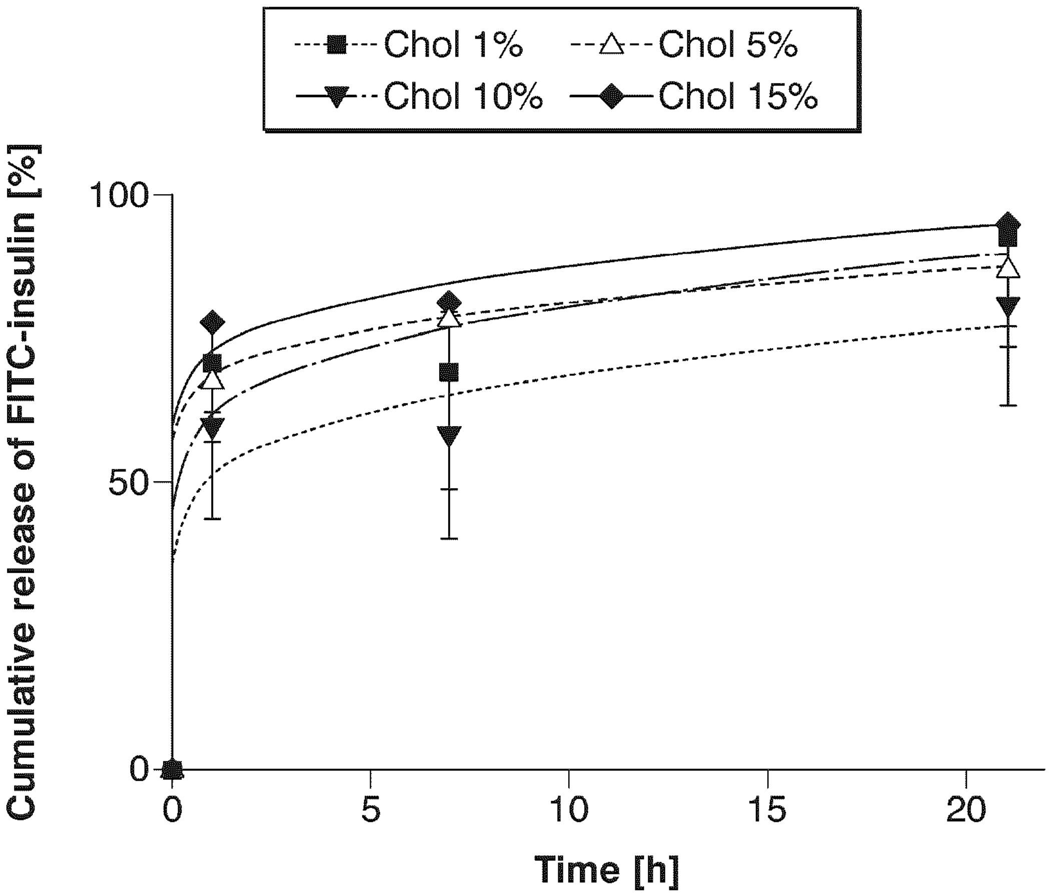
Chol 1%
Chol 5%
Chol 10%
Chol 15%
Cumulative release of FITC-insulin [%]
100
50
0
0
5
10
15
20
Time [h]

FIG. 7

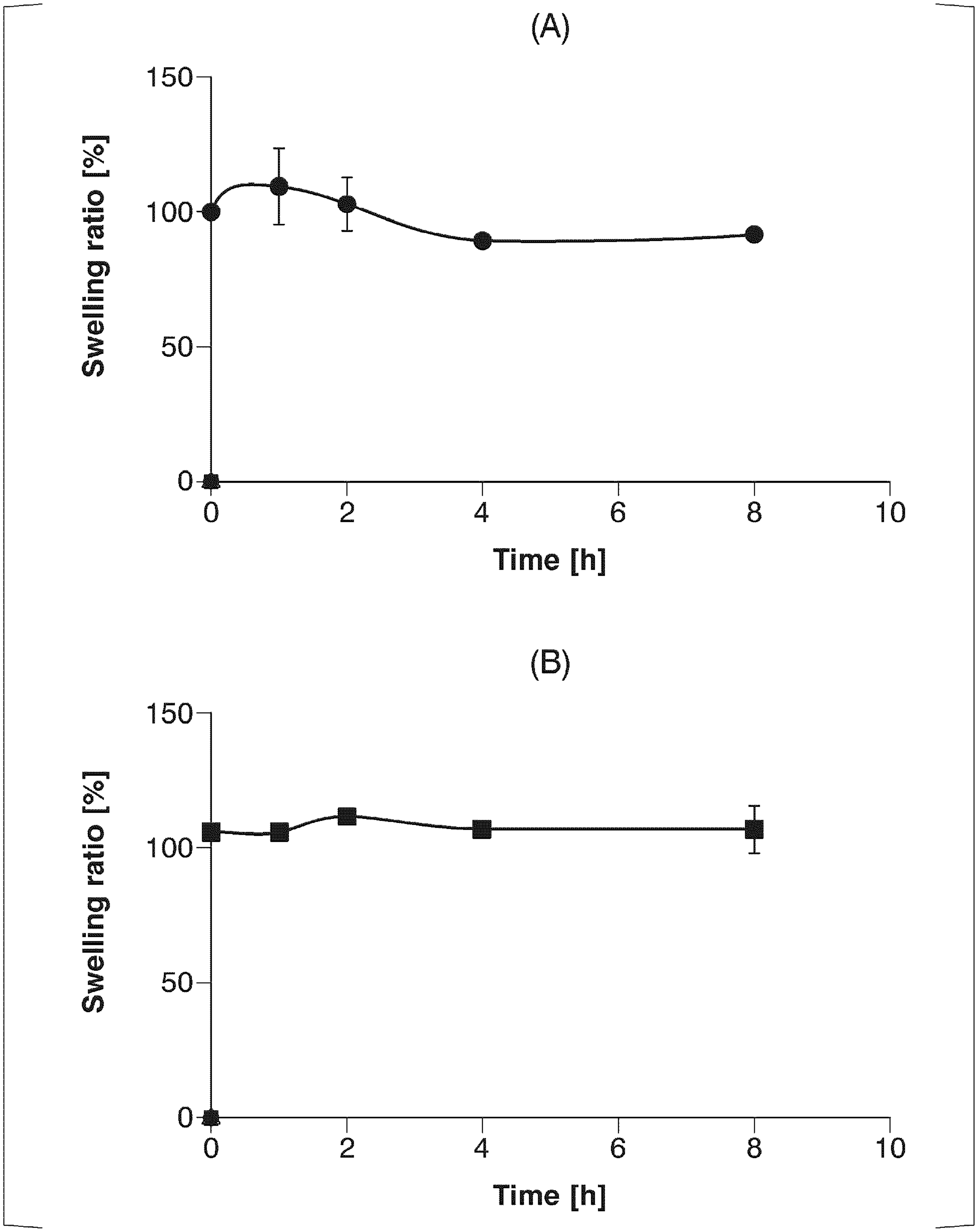
(A)
150
100
50
0
Swelling ratio [%]
0
2
4
6
8
10
Time [h]
(B)
150
100
50
0
Swelling ratio [%]
0
2
4
6
8
10
Time [h]

## INTERNATIONAL SEARCH REPORT

International application No.
**PCT/JP2024/034816**

### A. CLASSIFICATION OF SUBJECT MATTER

***C08B 37/08***(2006.01)i; ***A61K 31/728***(2006.01)i; ***A61K 38/28***(2006.01)i; ***A61K 45/00***(2006.01)i; ***A61K 47/36***(2006.01)i; ***A61L 27/20***(2006.01)i; ***A61P 19/02***(2006.01)i
FI: C08B37/08 Z; A61K31/728; A61K38/28; A61K45/00; A61K47/36; A61L27/20; A61P19/02

According to International Patent Classification (IPC) or to both national classification and IPC

### B. FIELDS SEARCHED

Minimum documentation searched (classification system followed by classification symbols)

C08B37/08; A61K31/728; A61K38/28; A61K45/00; A61K47/36; A61L27/20; A61P19/02

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN), JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| A | WO 2008/136536 A1 (NATIONAL UNIVERSITY CORPORATION TOKYO MEDICAL AND DENTAL UNIVERSITY) 13 November 2008 (2008-11-13) claims | 1-18 |
| A | WO 2010/053140 A1 (NATIONAL UNIVERSITY CORPORATION TOKYO MEDICAL AND DENTAL UNIVERSITY) 14 May 2010 (2010-05-14) claims, examples 1-26 | 1-18 |
| A | WO 2022/108264 A1 (SNVIA CO., LTD.) 27 May 2022 (2022-05-27) claims, example 1 | 1-18 |
| A | WO 2021/086072 A1 (PUSAN NATIONAL UNIVERSITY INDUSTRY-UNIVERSITY COOPERATION FOUNDATION) 06 May 2021 (2021-05-06) claims, synthesis example 1 | 1-18 |

☐ Further documents are listed in the continuation of Box C.　　☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 November 2024** | **19 November 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/034816**

| Patent document cited in search report | Publication date (day/month/year) | Patent family member(s) | Publication date (day/month/year) |
|---|---|---|---|
| WO 2008/136536 A1 | 13 November 2008 | US 2010/0204102 A1 claims<br>JP 8-136536 A1<br>EP 2143446 A1 | |
| WO 2010/053140 A1 | 14 May 2010 | US 2011/0212901 A1 claims, examples 1-26<br>JP 10-53140 A1<br>EP 2360188 A1 | |
| WO 2022/108264 A1 | 27 May 2022 | US 2023/0416472 A1 claims, example 1<br>JP 2023-552710 A<br>KR 10-2022-0069220 A | |
| WO 2021/086072 A1 | 06 May 2021 | US 2022/0387664 A1 claims, synthetic example 1<br>JP 2023-501200 A<br>EP 4053164 A1<br>KR 10-2021-0052135 A<br>CN 114667300 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2023166346 A
- JP 2023204796 A
- WO 2010053140 A
- WO 2004046200 A
- WO 2003087019 A
- JP 2021123597 A
- JP 2022013861 A
- JP 2022044579 A


### Non-patent literature cited in the description


- **ANDERSON et al.** *Analytical Chemistry*, 2003, vol. 75, 4279-4291
- **WYATT**. *Analytica Chimica Acta*, 1993, vol. 272 (1), 1-40